# EUROPEAN PATENT APPLICATION

(11) **EP 4 105 323 A1**
(43) Date of publication of application: **21.12.2022**
(21) Application number: 21179434.2
(22) Date of filing: 15.06.2021
(51) Int. Cl.: C12N 9/06, A23L 33/195

(54) **NEW MICROBIAL DIAMINE OXIDASE DERIVED FROM YARROWIA LIPOLYTICA FOR THE DEGRADATION OF BIOGENIC AMINES**

(71) Applicant: Universität Hohenheim, 70599 Stuttgart (DE)
(72) Inventor: FISCHER, Lutz, 73274 Notzingen (DE); KETTNER, Lucas, 70619 Stuttgart (DE); SEITL, Ines, 73760 Ostfildern (DE); LUTZ-WAHL, Sabine, 73230 Kirchheim (DE)
(74) Representative: Müller-Boré & Partner Patentanwälte PartG mbB

(57) **Abstract**

The present invention relates to a specific diamine oxidase (DAO) enzyme derived from the yeast *Yarrowia lipolytica* PO1f for use in medicine, in particular for use in the prevention or treatment of a condition or disease that is associated with increased levels of biogenic amines, uses of said enzyme and respective methods for the production of biogenic amine-depleted products, as well as functional foods and dietary supplement comprising said enzyme.

SEQUENCE LISTING

<110>  Universität Hohenheim

<120>  New microbial diamine oxidase derived from Yarrowia lipolytica
       for the degradation of biogenic amines

<130>  H 3901EU - kl

<160>  11

<170>  PatentIn version 3.5

<210>  1
<211>  671
<212>  PRT
<213>  Yarrowia lipolytica

<400>  1

Met Thr Pro His Pro Phe Asp Gln Leu Ser Val Gln Glu Met Glu Ser
1               5                   10                  15

Val Val Arg Val Val Lys Ser Asn His Ser Gly Lys Ser Leu His Leu
            20                  25                  30

Lys Ser Ile Gly Thr Glu Glu Pro Pro Lys Ala Leu Met Ala Pro Phe
        35                  40                  45

Leu Ala Ala Lys Arg Ala Gly Lys Asn Pro Val Pro Pro Pro Arg Ile
    50                  55                  60

Ala His Val Ile Tyr Tyr Val Leu Glu Asp Lys Leu Val Asn Gln Cys
65                  70                  75                  80

Trp Val Asp Val Pro Ser Ala Lys Val Val Lys Ser Glu Val Leu Lys
                85                  90                  95

Lys Gly Ile His Pro Pro Ile Asp Pro Trp Glu Ala Asn Glu Ala Phe
            100                 105                 110

Glu Ala Ala Phe Asp His Pro Leu Val Lys Asp Ala Ile Lys Lys Cys
        115                 120                 125

Gly Val Glu His Leu Ile Asp Asn Leu Thr Ile Asp Gly Trp Met Tyr
    130                 135                 140

Gly Cys Asp Ser Glu Ile Asp Met Pro Arg Tyr Leu Gln Met Leu Val
145                 150                 155                 160

Tyr Cys Arg Asp Pro Lys Thr Asn His Gln Asp Ser Asn Met Tyr Ala
                165                 170                 175

Phe Pro Val Pro Phe Val Pro Val Tyr Asp Val Leu Glu Lys Lys Leu
            180                 185                 190

Val Arg Val Asp Tyr Cys Ala Thr Gly Gly Asp Asp Asp Asp Ala Ala
        195                 200                 205

Val Glu Gly Val Gly Asn Tyr Asp Thr Arg Pro Glu Gly Lys Asn Cys
    210                 215                 220

Ile Glu His Cys Val Thr Asn Asp Tyr Leu Pro Glu Leu Gln Asp Lys
225                 230                 235                 240

Met Arg Thr Asp Leu Lys Pro Tyr Asn Val Leu Gln Pro Glu Gly Pro
                245                 250                 255

Ser Tyr His Ile Asp Ser Asp Gly Tyr Ile Asn Trp Gln Lys Trp His
            260                 265                 270

Phe Lys Val Gly Phe Thr Pro Arg Glu Gly Leu Val Ile His Asp Val
        275                 280                 285

His Tyr Asp Gly Arg Ser Thr Phe Tyr Arg Leu Ser Met Ser Glu Met
    290                 295                 300

Ala Val Pro Tyr Ala Asp Pro Arg Pro Pro Leu His Arg Lys Met Ala
305                 310                 315                 320

Phe Asp Phe Gly Asp Cys Gly Gly Gly Lys Cys Ala Asn Glu Leu Thr
                325                 330                 335

Leu Gly Cys Asp Cys Leu Gly Thr Ile Arg Tyr Phe Asp Gly Asn Val
            340                 345                 350

Cys Asp Pro Glu Gly Asn Val Phe Thr Arg Lys Asn Val Ile Cys Met
        355                 360                 365

His Glu Gln Asp Asp Gly Ile Gly Trp Lys His Thr Asn Tyr Arg Thr
    370                 375                 380

Asp Val Val Ala Ile Thr Arg Arg Arg Ile Leu Val Leu Gln Thr Ile
385                 390                 395                 400

Leu Thr Val Gly Asn Tyr Glu Tyr Ile Phe Ala Trp His Phe Asp Gln
                405                 410                 415

Ser Ala Gly Ile Gln Leu Glu Ile Arg Ala Thr Gly Ile Val Ser Thr
            420                 425                 430

Gln Leu Ile Asp Ala Gly Lys Lys Ser Lys Phe Gly Thr Ile Val Ser
        435                 440                 445

Pro Gly Val Met Ala Ala Ser His Gln His Ile Phe Asn Val Arg Met
    450                 455                 460

Asp Pro Ala Ile Asp Gly His Gln Asn Thr Val Val Val Asn Asp Thr
465                 470                 475                 480

Val Ala Leu Pro Trp Asp Ala Lys Asn Pro His Gly Ile Ala Phe Glu
                485                 490                 495

Asn Thr Lys Thr Pro Ile Glu Lys Ser Cys Tyr Leu Asp Ser Asp Ile
            500                 505                 510

Gln Lys Asn Arg Tyr Leu Lys Ile Cys Asn Glu Asn Lys Ile Asn Pro
        515                 520                 525

Ile Ser Gly Asn Pro Val Gly Tyr Lys Ile Gly Gly Leu Ala Thr Ala
    530                 535                 540

Met Leu Tyr Ala Gln Pro Gly Ser Val Ser Arg Asn Arg Ala Ala Phe
545                 550                 555                 560

Ala Thr His His Tyr Trp Val Thr Lys Tyr Lys Asp Gln Glu Leu Phe
                565                 570                 575

Ala Gly Gly Val Trp Thr Asn Gln Ser Ala Asn Glu Val Gly Gly Val
            580                 585                 590

Gln Asp Ala Val Ala Arg Asn Glu Asn Val Arg Asn Asp Asp Val Val
        595                 600                 605

Leu Trp His Ser Phe Gly Leu Thr His His Pro Arg Val Glu Asp Phe
    610                 615                 620

Pro Val Met Pro Cys Glu Ile Met Lys Ile His Leu Ser Pro Asn Asp
625                 630                 635                 640

Phe Phe Thr Gly Asn Pro Ser Val Asp Val Pro Lys Ser Asn Gln Thr
                645                 650                 655

Phe Asn Arg Ser Val Glu Val Lys Asp Cys Arg Ser Cys Lys Ile
            660                 665                 670

<210>  2

<211>  2016
<212>  DNA
<213>  Yarrowia lipolytica

<400>  2
atgactcccc accctttcga tcagctctcc gttcaggaga tggagagcgt tgtgcgagtg      60

gtcaagtcca accattcggg caagtctctt cacctcaagt ccattggcac cgaggagcct     120

cccaaggcgc tgatggctcc tttccttgca gccaagcgag ctggcaagaa ccccgttccc     180

ccccctcgaa tcgcacatgt catctactat gttctggagg acaagttggt gaaccagtgc     240

tgggtcgatg ttccttccgc caaggttgtc aagtccgagg tgctcaagaa gggcatccat     300

cctcccattg atccctggga ggccaacgag gccttcgagg ccgcctttga ccatcctctg     360

gtcaaggacg ccatcaagaa gtgcggcgtg gagcatctga tcgacaacct cacaattgac     420

ggctggatgt acggctgtga cagcgagatt gacatgcccc ggtacctgca gatgctggtc     480

tactgccgag atcccaagac caaccaccag gactccaaca tgtacgcctt ccccgttccg     540

tttgttcctg tctacgacgt gctggagaag aagctcgttc gagtcgacta ttgcgccacc     600

ggtggagacg atgacgacgc tgccgtcgag ggcgttggca actacgacac ccgacccgag     660

ggaaagaact gcatcgagca ctgtgtcacc aacgactatc ttcccgagct tcaggacaag     720

atgcgaaccg acctcaagcc ctacaacgtg ttgcagcccg agggtccctc ttaccacatt     780

gacagtgacg gctacatcaa ctggcaaaag tggcacttca aggtcggatt cactccccga     840

gagggtctgg tgatccacga tgtccactac gacggccgat ccaccttcta ccgactgtcc     900

atgtccgaga tggcggttcc ctacgccgat ccccggcccc ctctgcaccg aaagatggcg     960

tttgatttcg gcgactgtgg aggaggaaag tgcgccaacg agctgactct gggctgcgac    1020

tgtcttggta ccatccgata ctttgacggc aacgtgtgcg accccgaggg caacgtgttc    1080

acccgaaaga acgtcatctg tatgcacgag caggatgacg gtatcggctg gaagcacact    1140

aactaccgaa ccgacgtggt tgccatcacc cgacgacgaa ttctggttct gcagaccatt    1200

ctgaccgtgg gcaactacga gtacatcttt gcctggcact ttgaccagtc tgccggaatc    1260

cagctggaga tccgagccac cggaatcgtc tccacccagc ttatcgacgc cggcaagaag    1320

tccaagtttg gcaccattgt ctctcccgga gtcatggccg cctctcacca gcacattttc    1380

aacgtgcgaa tggaccctgc cattgacggc catcagaaca cagttgtggt caacgacacc    1440

gttgctctgc cctgggacgc caagaacccc catggaatcg cctttgagaa caccaagacc    1500

cccatcgaga agtcgtgcta cctggactcg gacatccaga agaaccggta cctcaagatc    1560

tgcaacgaga acaagatcaa ccccatctcc ggcaaccccg tgggctacaa gattggaggt    1620

ctggccaccg ccatgctgta cgctcagccc ggctccgttt cgcgaaaccg agccgccttt    1680

gccacccacc actactgggt caccaagtac aaggaccagg agctgtttgc cggcggcgtg    1740

tggaccaacc agtctgccaa cgaggtcggc ggagtccagg atgccgttgc ccgaaacgag 1800

aacgtgcgaa acgacgacgt ggttctctgg cactcctttg gtctcactca ccacccccga 1860

gtcgaggact tccccgtcat gccttgcgag attatgaaga tccatctgtc gcccaacgat 1920

ttcttcaccg gcaacccttc tgttgacgtg cccaagtcca accagacttt caatcgatcc 1980

gtcgaggtca aggactgtcg atcttgcaag atctag 2016

<210> 3
<211> 2091
<212> DNA
<213> Yarrowia lipolytica

<400> 3
atgcacagac tatcacaact agctacacaa acaaccgcgg ccaccatcac cgcaggtcat 60

cctcttgatc ctctctctcc ctccgaaatc gaacatgccg cttccatcgt caaatcgcag 120

atgcgagact cgtctccgta ccggttcaat ctcatcacgc tgatcgagcc gcccaaggcc 180

gagcttcttg cgtgggaggc gtcgccttcc tcggtggcca aacctcctcg acgagcggag 240

gtcgtactcg ttgtgagagg caagaagggc gtcaccgagg gccgggtctg tctcaccggc 300

tcaaaggtgc tttcgtggtc cgaaattgag ggcgtccagc ctattcttac cgttgacgac 360

ctccaaaagg tcgaggaaat tgtgcgacag gaccccgaag tcatcaagca gtgcaaactc 420

attggagttg acaacatgtc ccaggtgtac tgtgatccct ggactattgg ctatgacgaa 480

agatggggtg ccgaacgacg tctacagcag gcgttcctct acttccgagc ccaccaggac 540

gactcccagt actcccatcc tcttgacttc actccaatct acgacgccac ggagcagaaa 600

gtcatcttta ttgatattcc tcccgttcgt cgacctctct ccaagcttaa gaattccaac 660

ttcaaccctc aggatatctc caagactacc ggttacagag acgtgaagcc catcgacgtg 720

tctcagcccg aaggagtcaa cttcaagatg accggtcgaa tcatggagtg gcagggattc 780

cgatttcacg tgggattcaa ctacagagag ggaattgtgc tgtctcagat ctctttcaac 840

gaccatggta accagcgaaa catgttccat cgtctctctc tcgttgaaat ggttgttccc 900

tacggaaacc ccgagcaccc tcaccagcga aagcacgcct ttgatctggg agagtacgga 960

gccggtctca tgaccaatcc tctttccctc ggatgtgatt gcaagggagt cattcactac 1020

cttgatgcgc acttttccga cgccgaagga aagcctctca ctgttcccaa cgctgtgtgt 1080

atccacgagg aggacaatgg tctgcttttc aagcactctg acttccgaga cgagttccag 1140

acttcgatcg tcactcgagc taccaagctc attttgtcgc agattttcac cgccgcaaac 1200

tacgaatact gcgtctactg gattttccac caggacggta ccatccagct ggagattaag 1260

ctcaccggca tcctcaacac tttcccctgc aatcccggag aagatttgca tggctggggc 1320

acagaggtgt accctggtac caacgcccac aaccatcagc atctcttttc tctgcgaatc 1380

catcctgcca ttgattccca gctgcatcca ggtaattctg tggcaatggt ggacgctgag 1440

cggtcaccct tcccgcctgg acacccagaa aacctgcacg gcaatggttt ccggcccaag 1500

cgaacggtct tcaacaaccc gatcgaggct atgacggatt atgatggtaa tacatcgcga 1560

acttgggact ttttcaaccc gaagtccatc aaccagtact ccaagaagcc cgcttcttac 1620

aagctggtgt ctcgtgagtg ccctcctctg cttcctcagc ctggtggact ggtttggaac 1680

cgagctggtt ttgcacgaca ccatatgcat gttgttccgt atgtggacgg ccagctgtac 1740

cctgctggac ggtttgtttg ccagacaagt ggaaagccct caaggggtct ccccgagtgg 1800

attgagcagt gcggagagaa ggccaatatc aacgataccg atgttgttgc ctatcacact 1860

tttggtctga cccatttccc tgctcccgag gacttccctt tgatgcctgc cgagcccatg 1920

actctacttt tgcgacccag aaacttcttc ctgcagaacc cggctttgga cgtgcctcct 1980

tcgcatgctc gaaccaccac agaggcccag gctgcttccg gtgctaaggt tgtctctctt 2040

accgacaagg tgtcgcagct ggctttcaat aagtcgtgct gttccaagta g 2091

<210> 4
<211> 24
<212> DNA
<213> Artificial sequence

<220>
<223> Forward primer for the amplification of dao-1 from the genomic
      DNA of Y. lipolytica PO1f

<400> 4
atgactcccc accctttcga tcag 24

<210> 5
<211> 28
<212> DNA
<213> Artificial sequence

<220>
<223> Reverse primer for the amplification of dao-1 from the genomic
      DNA of Y. lipolytica PO1f

<400> 5
ctagatcttg caagatcgac agtccttg 28

<210> 6
<211> 27
<212> DNA
<213> Artificial sequence

<220>
<223> Forward primer for the second amplification of dao-1 and addition
      of restriction sites

<400> 6
cttgcgcgca tgactcccca ccctttc 27

<210>  7
<211>  27
<212>  DNA
<213>  Artificial sequence

<220>
<223>  Reverse primer for the second amplification of dao-1 and addition
       of restriction sites

<400>  7
ggcgctagcc tagatcttgc aagatcg                                          27


<210>  8
<211>  32
<212>  DNA
<213>  Artificial sequence

<220>
<223>  Forward primer for the amplification of dao-2 from the genomic
       DNA of Y. lipolytica PO1f and addition of restriction sites

<400>  8
gaagcgcgca tgcacagact atcacaacta gc                                    32


<210>  9
<211>  27
<212>  DNA
<213>  Artificial sequence

<220>
<223>  Reverse primer for the amplification of dao-2 from the genomic
       DNA of Y. lipolytica PO1f and addition of restriction sites

<400>  9
caagctagcc tacttggaac agcacga                                          27


<210>  10
<211>  25
<212>  DNA
<213>  Artificial sequence

<220>
<223>  Forward sequencing primer for dao genes

<400>  10
ctaaagatgt tgatctcctt gtgcc                                            25


<210>  11
<211>  21
<212>  DNA
<213>  Artificial sequence

<220>
<223>  Reverse sequencing primer for dao genes

<400>  11
cctctgggcc gaatacaaca c                                                21

## Description

The present invention relates to a specific diamine oxidase (DAO) enzyme derived from the yeast *Yarrowia lipolytica* PO1f for use in medicine, in particular for use in the prevention or treatment of a condition or disease that is associated with increased levels of biogenic amines, uses of said enzyme and respective methods for the production of biogenic amine-depleted products, as well as functional foods and dietary supplement comprising said enzyme.

Biogenic amines such as histamine are especially found in foods that undergo a fermentation process due to the presence of microorganisms producing L-histidine decarboxylase. L-histidine decarboxylase (EC 4.1.1.22) generates histamine through the decarboxylation of the precursor L-histidine during the fermentation or storage of the food. Histamine exhibits a multitude of physiological functions in the human, acting as an important hormone and neurotransmitter. Therefore, the consumption of large amounts of exogenous histamine in food can cause serious poisoning with various physiological symptoms, such as vomiting, diarrhea or hypotension. Other biogenic amines, such as tyramine, putrescine or cadaverine, are also frequently found in foods and can also cause toxicological effects in the human body. The consumption of moderate or even small amounts of histamine can also cause adverse allergy-like reactions in some susceptible individuals. It is estimated that around 1 % of the total population is intolerant towards histamine and, thereby, susceptible to minor dosages. This intolerance seems to derive from a disbalance between the amount of histamine ingested and the activity of the histamine degrading enzyme DAO (EC 1.4.3.22) available in the small intestine. This enzyme degrades histamine by oxidative deamination, resulting in the reaction products (imidazol-4-yl)acetaldehyde, hydrogen peroxide and ammonia.

There is currently no real treatment for the intolerance against biogenic amines such as histamine. People who are affected can administer a dietary supplement containing porcine DAO that is supposed to support the endogenous DAO in the small intestine. However, it has been shown that the activity required for a satisfactory histamine reduction is considerably larger than expected and that an alternative to the porcine DAO formulation used currently has to be found.

Specifically, DAO extracted from porcine kidney is commercially available in a dietary supplement and has already been investigated in clinical trials for its efficacy. However, it has recently been shown that this enzyme cannot be extracted and administered in sufficient quantities to achieve satisfactory histamine depletion. In particular, kinetic studies on porcine kidney DAO showed substrate inhibition by histamine concentrations greater than 56 mg/L (0.5 mM). The stability of free porcine DAO was tested in simulated intestinal fluid and showed a half-life of approximately 19 minutes. For the *in vitro* reduction of about 90% of histamine, a total of 50 nanokatal (nkat) free porcine DAO was required. This corresponded to the amount of enzyme isolated from about 100 g of pig kidney. The dietary supplement, containing a pig kidney extract did not show DAO activity. Instead, the histamine used in the experiment (0.75 mg) was apparently reduced by the adsorption to a capsule component by 18.9 ± 2.3% within 5 h. Although the capsule preparation retained its overall structure and shape for at least 90 min in simulated gastric fluid, the apparent histamine reduction was significantly reduced to 12.1 ± 2.3% (P ≤ 0.05). Thus, an alternative to porcine DAO is urgently needed to provide adequate supplementation for histamine-intolerant individuals.

In this context, microbial DAOs can be easily produced in large quantities in suitable expression systems. For this purpose, some microbial DAOs have been studied and characterized in the art. Microbial DAOs exhibit varying properties resulting in certain advantages and disadvantages with regard to the degradation of biogenic amines from food. For example, using a histamine oxidase from *Arthrobacter crystallopoietes* KAIT-B-007, histamine can be degraded with a high kinetic efficiency. However, this enzyme, as well as, for example, an amine oxidase from *Aspergillus niger,* is not suitable to degrade the biogenic amines putrescine, cadaverine or spermidine.

Accordingly, the technical problem underlying the present invention is the provision of improved means for the degradation of a broad range of biogenic amines *in vitro* and *in vivo.*

The solution to the above technical problem is achieved by the embodiments characterized in the claims.

In particular, in one aspect, the present invention relates to an enzyme having diamine oxidase activity for use in medicine, wherein said enzyme comprises
(i) the amino acid sequence of SEQ ID NO: 1; or
(ii) an amino acid sequence having at least 70% sequence identity to SEQ ID NO: 1 and having diamine oxidase activity.

In preferred embodiments, the above enzyme consists of
(i) the amino acid sequence of SEQ ID NO: 1; or
(ii) the amino acid sequence having at least 70% sequence identity to SEQ ID NO: 1 and having diamine oxidase activity.

In a specific embodiment, the above enzyme consists of the amino acid sequence of SEQ ID NO:1.

As used herein, the term "enzyme having diamine oxidase activity" relates to an enzyme that catalyzes the oxidation of biogenic amines according to the general reaction

R-CH₂-NH₂ + H₂O + O₂ → R-CHO + NH₃ + H₂O₂.

The enzyme having diamine oxidase activity used in the present invention is either (i) the *Yarrowia lipolytica* PO1f diamine oxidase-1 (DAO-1) having the amino acid sequence of SEQ ID NO: 1, the advantageous use of which in the degradation of biogenic amines has been discovered in the present invention, or (ii), in the case of an enzyme having at least 70% sequence identity to SEQ ID NO: 1 and having diamine oxidase activity, a respective DAO derived therefrom.

The term "enzyme comprising an amino acid sequence having at least 70% sequence identity to SEQ ID NO: 1 and having diamine oxidase activity" relates to polypeptides that can comprise any number of amino acid substitutions, additions, or deletions with respect to the amino acid sequence of SEQ ID NO: 1, e.g. 1, 2, 3, 4, 5, 6, 7, 8, 9, 10, or any number up to 200 (*i.e*., any integer n, wherein 1 ≤ n ≤ 200), amino acid substitutions, additions, or deletions with respect to the amino acid sequence of SEQ ID NO: 1, provided that the resulting polypeptides fulfil the requirement of having at least 70% sequence identity to SEQ ID NO: 1 and retain biological activity of a diamine oxidase. In this context, the term "retains the biological activity of a diamine oxidase" as used herein relates to polypeptides that have at least 5%, at least 10%, at least 15%, at least 20%, at least 25%, at least 30%, at least 35%, at least 40%, at least 45%, at least 50%, preferably at least 55%, at least 60%, at least 65%, at least 70%, at least 75%, at least 80%, at least 82%, at least 84%, at least 86%, at least 88%, at least 90%, at least 91%, at least 92%, at least 93%, at least 94%, at least 95%, at least 96%, at least 97%, at least 97.5%, at least 98%, at least 98.5%, at least 99%, at least 99.5%, 100%, or more than 100% of the activity of the *Y. lipolytica* PO1f DAO-1 having the amino acid sequence of SEQ ID NO: 1, as determined in a standard diamine oxidase activity assay known in the art.

While the number of amino acid substitutions, additions, or deletions is generally only limited by the above proviso concerning the sequence identity, biological activity of the resulting polypeptide, and absolute number of amino acid substitutions, additions or deletions, it is preferable that the resulting polypeptide has at least 72%, at least 74%, at least 76%, at least 78%, at least 80%, at least 82%, at least 84%, at least 86%, at least 88%, at least 90%, at least 91%, at least 92%, at least 93%, at least 94%, at least 95%, at least 95.5%, at least 96%, at least 96.5%, at least 97%, at least 97.5%, at least 98%, at least 98.2%, at least 98.4%, at least 98.6%, at least 98.8%, at least 99%, at least 99.1 %, at least 99.2%, at least 99.3%, at least 99.4%, at least 99.5%, at least 99.55%, at least 99.6%, at least 99.65%, at least 99.7%, at least 99.75%, at least 99.8%, at least 99.85%, at least 99.9%, at least 99.91%, at least 99.92%, at least 99.93%, at least 99.94%, at least 99.95%, at least 99.96%, at least 99.97%, at least 99.98%, or at least 99.9% sequence identity to SEQ ID NO: 1.

Means for determining the sequence identity of an amino acid sequence to a reference sequence are known in the art.

In specific embodiments, the above enzyme is for use in a method of preventing or treating a condition or disease that is associated with increased levels of biogenic amines in a subject, preferably a mammalian subject, more preferably a human subject.

In this context, the term "increased levels of biogenic amines" as used herein refers to increased levels of biogenic amines *in vivo,* e.g. in the small intestine, where biogenic amines first accumulate before passing into the bloodstream, or in the blood of the subject. Means for determining the level of biogenic amines in a subject are not particularly limited and are known in the art.

Increased *in vivo* levels of biogenic amines can result from pathological conditions in the body, e.g. dysregulated immune functions as in allergic reactions, or can result from the ingestion of biogenic amines by the subject, e.g. by consuming foodstuffs containing high levels of biogenic amines.

In specific embodiments, the condition or disease that is associated with increased levels of biogenic amines is selected from the group consisting of allergies, acute and chronic allergic diseases, allergic reactions, allergy-like reactions, itching (pruritus), diarrhea, redness (erubescence), vomiting (emesis), acute and chronic biogenic amine poisoning, hypotonia, difficulty of breathing, biogenic amine intolerance, anaphylaxis, anaphylactic shock, acute and chronic urticaria, asthma, hay fever, allergic rhinitis, allergic conjunctivitis, headache, migraine, atopic dermatitis, mastocytosis, mast cell activation syndrome (MCAS), pre-eclampsia, hyperemesis gravidarum, pre-term labor, peptic ulcers, acid reflux, sepsis, fibromyalgia, chronic fatigue syndrome, and spondylitis.

The term "biogenic amines" as used herein relates to any amine compound that is a constituent of, secreted by or a metabolite of a plant, animal, fungus or microorganism, provided that said amine can have an adverse effect on a subject. However, in preferred embodiments, the biogenic amine is selected from the group consisting of histamine, tyramine, putrescine, cadaverine, agmatine, spermidine, and tryptamine, preferably from the group consisting of histamine, tyramine, putrescine, and cadaverine. In specific embodiments, the biogenic amine is histamine.

In a related aspect, the present invention relates to a method of preventing or treating a condition or disease that is associated with increased levels of biogenic amines in a subject, comprising the step of administering an enzyme having diamine oxidase activity to the subject, wherein said enzyme comprises
(i) the amino acid sequence of SEQ ID NO: 1; or
(ii) an amino acid sequence having at least 70% sequence identity to SEQ ID NO: 1 and having diamine oxidase activity.

In this aspect, all relevant definitions and limitations indicated above for the enzyme for use of the present invention apply in an analogous manner. In particular, the enzyme as such, the condition or disease that is associated with increased levels of biogenic amines in a subject, the subject, and the biogenic amines are as defined above.

In this context, dosages, dosage regimens, administration modes and suitable formulations for the enzymes used in the present invention are not particularly limited and are known to the person skilled in the art and/or can be easily determined by the person skilled in the art.

In a further aspect, the present invention relates to the use of an enzyme having diamine oxidase activity in the production of a biogenic amine-depleted product, wherein said enzyme comprises
(i) the amino acid sequence of SEQ ID NO: 1; or
(ii) an amino acid sequence having at least 70% sequence identity to SEQ ID NO: 1 and having diamine oxidase activity.

In this aspect, all relevant definitions and limitations indicated above for the enzyme for use of the present invention apply in an analogous manner. In particular, the enzyme as such and the biogenic amines are as defined above.

Means of using the enzyme used in the present invention in the production of a biogenic amine-depleted product are not particularly limited and are known in the art. Respective means include for example the step of contacting (i) a biogenic amine-containing product, and/or (ii) a biogenic amine-containing intermediate product of said product, with said enzyme under conditions and for a duration of time suitable to degrade a biogenic amine present in said product and/or in said intermediate product of said product. Respective conditions and or durations of time are not particularly limited and are known to the person skilled in the art and/or can be easily determined by the person skilled in the art. These include for example the incubation of the product or intermediate product thereof with the enzyme in an amount providing an enzyme activity of 0.1 nkat/mL or 0.1 nkat/mg of the product or intermediate product thereof within at most 5 h at a temperature of 37 ± 1 °C and in a pH range of pH 6 to 8.

The term "biogenic amine-depleted product" as used herein refers to a product whose biogenic amine content has been reduced with respect to its original biogenic amine content. In specific embodiments, the biogenic amine content has been reduced by at least 50%, at least 55%, at least 60%, at least 65%, at least 70%, at least 75%, at least 80%, at least 85%, at least 90%, at least 95%, at least 95.5%, at least 96%, at least 96.5%, at least 97%, at least 97.2%, at least 97.4%, at least 97.5%, at least 97.6%, at least 97.7%, at least 97.8%, at least 97.9%, at least 98%, at least 98.1 %, at least 98.2%, at least 98.3%, at least 98.4%, at least 98.5%, at least 98.6%, at least 98.7%, at least 98.8%, at least 98.9%, at least 99%, at least 99.1 %, at least 99.2%, at least 99.3%, at least 99.4%, at least 99.5%, at least 99.6%, at least 99.7%, at least 99.8%, or at least 99.9%.

Products amenable to the above use according to the present invention are not particularly limited and include any products containing biogenic amines in which the reduction of biogenic amine content might be of interest. Preferably, the product is selected from the group consisting of foodstuffs and feedstuffs. In this context, the term "foodstuffs" as used herein refers to any solid or liquid comestibles, e.g. foods and drinks, that can be ingested by a human for nutritional and/or recreational purposes. Likewise, the term "feedstuffs" as used herein refers to any solid or liquid comestibles, e.g. feeds, that can be ingested by an animal for nutritional purposes. In specific embodiments, the above product is a foodstuff, selected from the group consisting of fermented foodstuffs, such as cheeses, sauerkraut, sausages, wine, chocolate, and yeast extracts, and fish and fish products, raw meats, and fresh milk.

In a related further aspect, the present invention relates to a method for the production of a biogenic amine-depleted product, comprising the step of contacting (i) a biogenic amine-containing product, and/or (ii) a biogenic amine-containing intermediate product of said product, with an enzyme having diamine oxidase activity under conditions and for a duration of time suitable to degrade a biogenic amine present in said product and/or in said intermediate product of said product, wherein said enzyme comprises
(i) the amino acid sequence of SEQ ID NO: 1; or
(ii) an amino acid sequence having at least 70% sequence identity to SEQ ID NO: 1 and having diamine oxidase activity.

In this aspect, all relevant definitions and limitations indicated above for the enzyme for use of the present invention and/or the use of the present invention apply in an analogous manner. In particular, the enzyme as such, the product, the term "biogenic amine-depleted product", and the biogenic amines are as defined above.

Respective conditions and or durations of time applicable in the methods of the present invention are not particularly limited and are known to the person skilled in the art and/or can be easily determined by the person skilled in the art. These include for example the incubation of the product or intermediate product thereof with the enzyme as defined above for the uses of the present invention.

In a further aspect, the present invention relates to a product obtained by the above method.

In this aspect, all relevant definitions and limitations indicated above for the enzyme for use of the present invention, the use of the present invention, and/or the method of the present invention apply in an analogous manner. In particular, the enzyme as such, the product, the term "biogenic amine-depleted product", and the biogenic amines are as defined above.

In a further aspect, the present invention relates to a functional food, dietary supplement, or pharmaceutical composition, comprising an enzyme having diamine oxidase activity, wherein said enzyme comprises
(i) the amino acid sequence of SEQ ID NO: 1; or
(ii) an amino acid sequence having at least 70% sequence identity to SEQ ID NO: 1 and having diamine oxidase activity.

In this aspect, all relevant definitions and limitations indicated above for the enzyme for use of the present invention apply in an analogous manner. In particular, the enzyme as such is as defined above.

In this context, the term "functional food" as used herein refers to any foodstuffs, e.g. any solid or liquid comestibles, e.g. foods and drinks, that are "functionalized" by the addition of the enzyme used in the present invention, *i.e*., that are modified to exhibit the additional effect of being able to reduce the levels of biogenic amines in a subject, preferably a human subject, upon ingestion of the functional food.

Further, the term "dietary supplement" as used herein refers to any manufactured product intended to supplement a subject's diet, preferably a human subject's diet, in the form of a pill, capsule, tablet, powder or liquid.

Particular foodstuffs, forms of dietary supplements, forms of pharmaceutical compositions, dosages, dosage regimens, and suitable formulations for the enzymes used in the present invention are not particularly limited and are known to the person skilled in the art and/or can be easily determined by the person skilled in the art.

In a final aspect, the present invention relates to the use of an enzyme having diamine oxidase activity in a functional food or in a dietary supplement, wherein said enzyme comprises
(i) the amino acid sequence of SEQ ID NO: 1; or
(ii) an amino acid sequence having at least 70% sequence identity to SEQ ID NO: 1 and having diamine oxidase activity.

In this aspect, all relevant definitions and limitations indicated above for the enzyme for use of the present invention. as well as for the functional food or dietary supplement of the present invention, apply in an analogous manner. In particular, the enzyme as such, the functional food, and the dietary supplement are as defined above.

As used herein, the term "comprising"/"comprises" expressly includes the terms "consisting essentially of"/"consists essentially of" and "consisting of"/"consists of", *i.e*., all of said terms are interchangeable with each other herein.

Further, as used herein, the term "about" represents a modifier of ± 10% of the specified value, preferably ± 7.5%, ± 5%, ± 3%, ± 2%, or ± 1% of the specified value. Thus, by way of example, the term "about 10" includes the ranges 9 to 11, 9.25 to 10.75, 9.5 to 10.5, 9.7 to 10.3, 9.8 to 10.2, and 9.9 to 10.1.

The diversity of enzymes found in microorganisms could have the potential of providing a competitive DAO with similar or even better attributes compared to e.g. porcine DOA as known in the art. If administered in a dietary supplement or generally used in the food industry, an advantage of microbial DAOs is the cost-effective and convenient enzyme production. Accordingly, the present invention identified *Yarrowia lipolytica* (*Y. lipolytica*) PO1f as a promising producer of an alternative microbial DAO. This yeast represents one of the yeast species most frequently found in raw milk and is also found in various types of cheese. It contributes to the development of flavor and aroma during the ripening of the cheese due to its proteolytic and lipolytic activity and is, therefore, a desirable and important constituent of the product. The yeast occurs naturally in cheese since contamination can happen at different production stages (raw milk, air, brine, contact with surfaces) and it grows well under the common environmental conditions in foods. However, *Y. lipolytica* was classified as an opportunistic pathogen in 2018 by the European Food Safety Authority and was given the qualified presumption of safety status only for production purposes. This means that the yeast should not be present in the final food as viable cells but is allowed to be used as an inactivated biomass as a novel food. *Y. lipolytica* was identified as a biogenic amine producer but was also suspected of being capable of degrading biogenic amines during the ripening of the food. However, the enzymes responsible for this degradation of biogenic amines had not been identified or described prior to the present invention.

According to the present invention, the bioreactor cultivation of the genetically modified *Yarrowia lipolytica* PO1f achieved a specific DAO activity of 1301 ± 54.2 nkat/g_{Protein}, which corresponded to a 93-fold increase in specific DAO activity compared with native DAO-1 production in *Y. lipolytica* PO1f and a 145-fold increase in specific DAO activity compared with DAO extraction from porcine kidney. Subsequent workup including chromatographic purification yielded a specific DAO activity of 4738 ± 31 nkat/g_{Protein}, which corresponded to a nearly 60-fold increase in specific DAO activity compared with extracted and purified DAO from porcine kidney. One liter of bioreactor cultivation generated approximately the same DAO activity obtained after extraction and partial purification from 5 kg of pig kidney. Moreover, the microbial production of DAO-1 requires much less work than extraction from pig kidney.

DAO-1 was investigated herein *in vitro* studies with regard to the efficiency of histamine degradation. The initial 150 mg/L (100%) of histamine used was degraded within 5 hours (37 °C) to 37.4 ± 0.65 mg/L (25 ± 1.7 %). Although compared to porcine DAO, DAO-1 according to the present invention has a lower affinity for the substrate histamine, DAO-1 showed similar satisfactory histamine degradation *in vitro.*

DAO-1 exhibits the peculiarity among microbial DAOs that, in addition to histamine, it also degrades other relevant biogenic amines such as tyramine, putrescine, cadaverine, agmatine, spermidine, and tryptamine. Since these biogenic amines are also present in fermented foods and, like histamine, have toxicological properties for humans, it is advantageous if they can be degraded simultaneously by an enzyme. DAO from the pig kidney is also able to degrade histamine, putrescine and cadaverine. However, the biogenic amine tyramine is not metabolized by this enzyme. Thus, the somewhat more efficient histamine degradation by DAO from the porcine kidney is outweighed by the advantages of the more efficient and simpler production of DAO-1 and the broad substrate spectrum.

In summary, the present invention provides uses of a *Yarrowia lipolytica* PO1f-derived diamine oxidase (DAO-1) that is advantageously and unexpectedly characterized by (i) superior specific activity, (ii) good *in vitro* degradation of biogenic amines such as histamine, and (iii) a broad substrate selectivity. Further, the DAO-1 enzyme used in the present invention can advantageously be produced in a much simpler, faster, more efficient and economic manner. Furthermore, the use of a yeast-derived DAO as opposed to e.g. animal-derived DAO poses less problems with respect to ethical aspects, as well as user acceptance and compliance.

The figures show:
Figure 1:
   (A) pHR*_axp_dao-1* plasmid for the integration of *dao-1* into the genome (*axp* locus) of *Y. lipolytica* PO1f by homologous recombination. (B) pHR*_axp_dao-2* plasmid for the integration of *dao-2* into the genome (axp locus) of *Y. lipolytica* PO1f by homologous recombination.
Figure 2:
   BLASTP amino acid sequence alignment of porcine DAO with the two putative DAOs DAO-1 (A) and DAO-2 (B) from *Y. lipolytica* PO1f.
Figure 3:
   Bioreactor cultivation of *Y*. *lipolytica* PO1f*_axp_dao-1* and the reference strain *Y. lipolytica* PO1f in YPD_{Ura} medium. Working volume: 0.8 L, 28 °C, pH 6.5.
Figure 4:
   Hydrophobic interaction chromatography purification of DAO-1. Column material: Toyopearl phenyl 650M (CV = 22 mL).
Figure 5:
   SDS-PAGE analysis of the DAO-1 purification by salt precipitation and hydrophobic interaction chromatography. Precision Plus Protein^{™} unstained protein standard 10-250 kDa (M); Crude extract after disruption (CE); Supernatant after 20 % (w/v) salt precipitation (S20 %); Suspended pellet after 60 % (w/v) salt precipitation (P60 %), HIC flow through (FT); HIC elution fractions (D2 - E7).
Figure 6:
   Native-PAGE analysis of the purified DAO-1. Marker (M): Serva native marker (21 - 720 kDa); 5 µg_{Protein} per lane; stained with Coomassie Brilliant Blue G-250 (C) and active (A) using histamine as substrate.
Figure 7:
   Influence of temperature on the maximal activity of DAO-1. DAO-1 activity measured with the DA-67 assay in PIPES buffer (25 mM; pH 7.2). Maximal DAO-1 activity (100 %) = 1.88 ± 0.01 nkat/mL.
Figure 8:
   Influence of the pH-value and buffer on the maximal activity of DAO-1. DAO-1 activity measured with the DA-67 assay at 37 °C. Maximal DAO-1 activity (100 %) = 1.84 ± 0.03 nkat/mL.
Figure 9:
   Residual activity of DAO-1 depending on buffer system (each 20 mM) and pH-value after 5 h at 37 °C. DAO-1 activity measured with the DA-67 assay at 37 °C under standard assay conditions. Residual activities of each data point are compared to the initially applied DAO-1 activity and given in percent (100 % = 0.156 ± 0.01 nkat/mL).
Figure 10:
   Michaelis-Menten kinetics of DAO-1. DAO-1 activity determined with the DA-67 assay in PIPES buffer (25 mM; pH 7.2) at 37 °C.
Figure 11:
   Michaelis-Menten kinetics of DAO-1 linearized according to Hanes-Woolf; enzyme activity determined with the DA-67 assay with histamine (0.5 mM - 50 mM) as substrate at 37 °C in PIPES buffer (25 mM; pH 7.2).
Figure 12:
   *In vitro* reduction of histamine in a food-relevant concentration (150 mg/L; 1.35 mM) using 0.1 nkat/mL of DAO-1 at 37 °C.

The present invention will be further illustrated by the following examples without being limited thereto.

### Examples

### Material and methods:

### Materials and reagents

Disodium hydrogen phosphate (Na₂HPO₄), ammonium sulfate ((NH₄)₂SO₄), 1,4-piperazinediethanesulfonic acid (PIPES), tris-hydrogen chloride (-HCl), histamine dihydrochloride, sodium chloride (NaCl), sodium hydroxide (NaOH), 2-propanol, D(+)-sucrose and hydrogen peroxide 30 % were purchased from Carl Roth GmbH (Karlsruhe, Germany). Sodium dihydrogen phosphate, sodium diethyldithiocarbamate, ortho-phosphoric acid (H₃PO₄) and thiamine chloride dihydrochloride were purchased from Merck (Darmstadt, Germany). Bovine serum albumin (BSA; modified Cohn Fraction V, pH 5.2) and Serva native marker (21-720 kDa) were purchased from Serva electrophoresis GmbH (Heidelberg, Germany). Bacto^{™} peptone, BD^{™} Difco^{™} Yeast nitrogen base and T4 DNA ligase were purchased from Thermo Fisher Scientific (Waltham, USA). Yeast Synthetic Drop-out Medium Supplements (without uracil, leucine and tryptophan), Antifoam 204, phenylmethylsulfonylfluoride (PMSF), histamine (analytically pure), cadaverine, putrescine dihydrochloride, spermidine trihydrochloride, tryptamine, tyramine hydrochloride and catalase (from *Micrococcus lysodeikticus;* 111700 U/mL) were purchased from Sigma-Aldrich (Merck) (St. Louis, USA). Agmatine dihydrochloride was purchased from Synthonix Inc. (Wake Forest, USA). Uracil was purchased from Alfa Aesar (Haverhill, USA). (10-(carboxymethyl-aminocarbonyl)-3,7-bis(dimethylamino) phenothiazine sodium salt (DA-67) was purchased from Fujifilm Wako Chemicals U.S.A. Corp (Richmond, USA). Horseradish peroxidase (Grade I) was purchased from AppliChem GmbH (Darmstadt, Germany). Q5^{®} High-Fidelity DNA polymerase and the restriction enzymes BssHII and *Nhe*I were purchased from New England Biolabs GmbH (Ipswich, USA). Precision Plus Protein^{™} unstained protein standard 10-250 kDa was purchased from Bio-Rad laboratories GmbH (Feldkirchen, Germany). The resin material Toyopearl Phenyl-650M was purchased from Tosoh Bioscience Inc. (San Francisco, USA).

### Strains and media

The genes for the putative DAOs and plasmid propagation were cloned in *Escherichia coli* XL-1, grown in lysogeny broth media with 100 µg/mL ampicillin.

*Yarrowia lipolytica* PO1f was obtained from the International Centre for Microbial Resources and cultivated in yeast extract peptone dextrose (YPD) media (10 g/L yeast extract, 20 g/L bacto^{™} peptone, 20 g/L glucose). Screening for positive transformants of the homologous recombinant integration of *dao-1* and *dao*-2 in *Y. lipolytica* PO1f was done on selective agar which contained 6.7 g/L yeast nitrogen base (BD^{™} Difco^{™}), 0.9 g/L CSM (Yeast Synthetic Drop-out Medium Supplements without uracil, leucine and tryptophan), 20 g/L glucose and 15 g/L agar.

### Cloning of the dao genes into the vector for homologous recombination (pHR)

Genomic DNA of *Y*. *lipolytica* PO1f was isolated by mechanical cell disruption using glass beads (0.5 - 0.7 mm in diameter) and subsequent DNA extraction in ROTI^{®}Phenol/Chloroform/Isoamyl as known in the art. *Y. lipolytica* PO1f was grown in 5 mL YPD in test tubes for 20 h at 30 °C, stirred at 180 rpm and was completely harvested for DNA isolation.

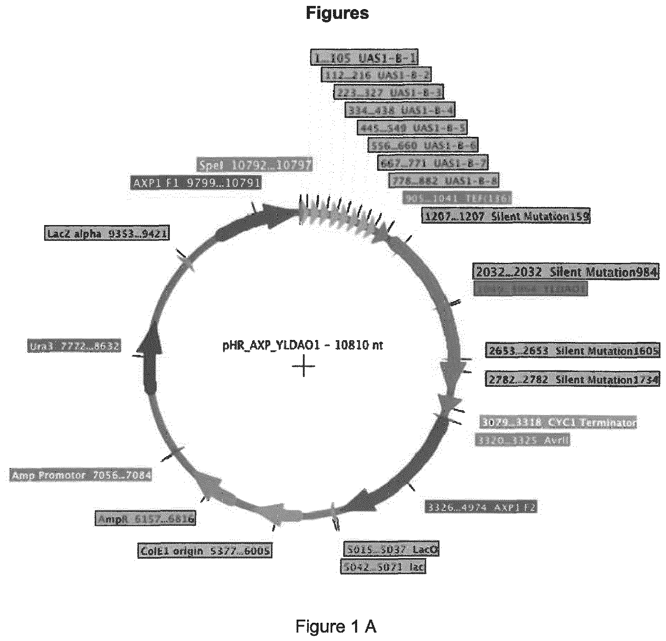

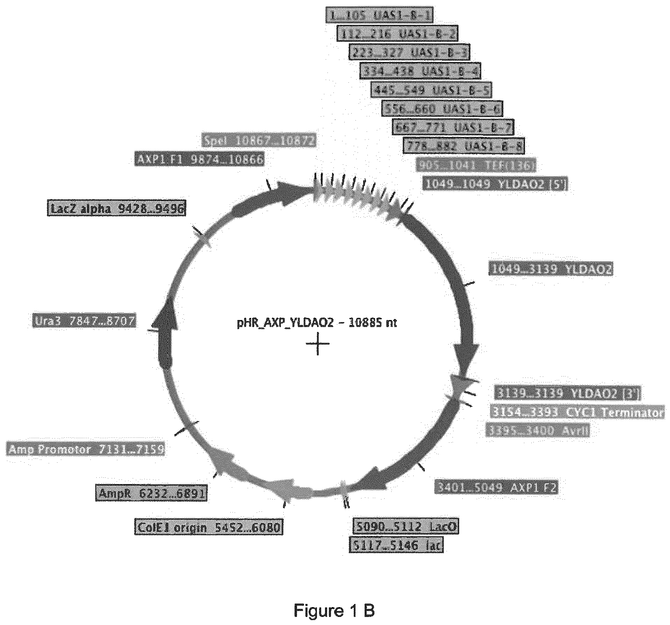

The genes coding for the two putative DAOs in *Y. lipolytica* PO1f (SEQ ID NOs: 2 (dao-1) and 3 (dao-2)) were amplified from genomic DNA by PCR using the Q5^{®} High-Fidelity DNA polymerase, according to the manufacturer's instructions. The primers used for the amplification of *dao*-1 from the genomic DNA of *Y*. *lipolytica* PO1f were (5'-ATGACTCCCCACCCTTTCGATCAG-3'; SEQ ID NO: 4) and (5'-CTAGATCTTGCAAGATCGACAGTCCTTG-3'; SEQ ID NO: 5). The primers for *dao*-1 were annealed at 69 °C for 30 s (35 cycles). The PCR product (2016 bp) was used directly for a second consecutive PCR amplification and restriction sites for *Bss*HII and *Nhe*I were added. The primers used for this amplification were (5'-CTTGCGCGCATGACTCCCCACCCTTTC-3'; SEQ ID NO: 6) and (5'-GGCGCTAGCCTAGATCTTGCAAGATCG-3'; SEQ ID NO: 7). The primers used for the amplification of *dao-2* (2145 bp) from genomic DNA introduced restriction sites for *Bss*HII (5'-GAAGCGCGCATGCACAGACTATCAC AACTAGC-3'; SEQ ID NO: 8) and *Nhe*I (5'- CAAGCTAGCCTACTTGGAACAGC ACGA-3'; SEQ ID NO: 9) directly into the PCR product. The primers for *dao-2* were annealed at 68 °C for 30s (35 cycles). The amplicons *dao-1_Bss*HII*_Nhe*I and *dao-2_Bss*HII*_Nhe*I obtained were purified (DNA clean & concentrator-25, Zymo Research, Irvine, USA) before they were used for *Bss*HII and *Nhe*I restriction digestion. The amplicons digested were purified using the GeneJET gel extraction kit (Thermo Fisher Scientific, Waltham, USA) after electrophoretic separation on a 1 % (w/v) agarose gel. The genes were ligated (T4 DNA ligase) into a *Bss*HII and *Nhe*I digested pHR_axp vector (Fig. 1), which comprises 1 kb flanking regions for the homologous recombinant integration into the acid extracellular peptidase (*axp*) locus of *Y. lipolytica* PO1f. Subsequently, chemically competent *Escherichia coli* XL-1 cells were transformed with the ligation approach by heat shock transformation. The cells were plated on lysogeny broth agar plates (100 µg/mL ampicillin) and grown overnight at 37 °C. Colonies were picked and cultivated in 5 mL lysogeny broth media (100 µg/mL ampicillin) at 37 °C for 16 h and stirred at 180 rpm. The plasmids pHR*_axp_dao-1* and pHR*_axp_dao-2* were isolated using the GeneJET plasmid miniprep Kit (Thermo Fisher Scientific, Waltham, USA), according to the manufacturer's instructions. The *dao* genes were analyzed by sequencing (Eurofins Genomics GmbH, Germany) using primers for the *axp* flanking regions (5'-CTAAAGATGTTGATCTCCTTGTG CC-3'; SEQ ID NO: 10) and (5'-CCTCTGGGCCGAATACAACAC-3'; SEQ ID NO: 11) as known in the art.

### Homologous recombinant integration of dao genes in Y. lipolytica PO1f

The genes for the putative DAOs *dao-1* and *dao-2* were homologously recombinantly integrated from the respective pHR vector (pHR*_axp_dao-1* and pHR*_axp_dao-2;* Fig. 1) into the *axp* locus on the genome of *Y. lipolytica* PO1f using the CRISPR-Cas9 system, as known in the art.

### Bioreactor cultivation of Y. lipolytica PO1f_axp_dao-1

Preliminary experiments showed that DAO-2 did not show enzyme activity towards histamine but only towards agmatine. Therefore, DAO-2 was not further investigated in the present invention.

*Y. lipolytica* PO1f*_axp_dao-1* and *Y. lipolytica* PO1f (negative control) were cultivated in the Multifors bioreactor system (1 L reactor volume, Infors HT) in a working volume of 800 mL. The bioreactors containing 720 mL of YPDura media (YPD media + 0.2 g/L uracil and 0.2 mL/L Antifoam 204) were inoculated with 80 mL preculture, which was cultivated in YPDura in 500 mL shaking flasks and incubated at 30 °C for 20 h and stirred at 115 rpm. The bioreactors were constantly aerated with 0.5 vvm and heated to 28 °C. The stirrer speed was stepwise increased (starting at 800 rpm) to keep the partial oxygen pressure pO₂ above 20 %. The pH was kept constant at 6.5 using 2 M H₃PO₄ and 2 M NaOH. The pO₂ and pH values during the bioreactor cultivations were monitored using the InPro6900 and 405-DPAS-SC-K8S pH sensors, respectively. Samples were taken constantly over the course of the bioreactor cultivation to monitor the optical density (OD₆₀₀), bio dry mass and glucose concentration in the media. A 15 mL sample was taken for the determination of the intracellular DAO-1 activity every 8 h. These samples were centrifuged (8000 g, 7 min, 4 °C) and washed twice with saline (0.9 % (w/v) NaCl), before the cell pellets were stored at -20 °C. The entire cell mass was harvested after 96 h of cultivation by centrifugation (6000 g, 10 min, 4 °C). The pellet was washed twice with saline and finally centrifuged at 8000 g, 15 min, 4 °C, before it was stored at -20 °C until further processed.

### Cell disruption and desalting for protein analysis and DAO activity determination

The yeast cells were thawed on ice and suspended (20 % (w/v) cell suspension) in PIPES buffer (25 mM, pH 7.2) containing 0.1 mM PMSF (dissolved in 2-propanol). The enzyme samples taken over the course of the bioreactor cultivation were mechanically disrupted using a French press (SLM Aminco FA-078, American Instrument Exchange, Haverhill, USA) in two consecutive steps at 1 kbar. The cell mass harvested at the end of the cultivation was disrupted using a high-pressure homogenizer (One Shot 20 KPSI, Constant Systems Limited, Daventry, UK) in two consecutive steps at 1.35 kbar. The cell debris was removed by centrifugation (8000 g, 5 min, 4 °C) after cell disruption. The samples taken over the course of the bioreactor cultivation for the determination of enzyme activity were desalted after disruption using PD-10 columns (GE Healthcare, Chicago, USA) against PIPES buffer (25 mM, pH 7.2), according to the manufacturer's instructions.

### Purification of DAO-1

The DAO-1 was purified by fractionated (NH₄)₂SO₄ precipitation and subsequent hydrophobic interaction chromatography (HIC). Therefore, 10 g of *Y. lipolytica* PO1f_*dao-1* cells were suspended and disrupted, as described above. Liquid (NH₄)₂SO₄ (4 M) was added dropwise (2 mL/min) to the cell-free extract to a final concentration of 20 % (v/v) and further equilibrated for 1 h on ice with stirring (350 rpm). Thereafter, the solution was centrifuged at 10,000 g for 10 min at 4 °C. The supernatant was precipitated again and the (NH₄)₂SO₄ concentration in the solution was increased from 20 % (v/v) to 60 % (v/v). The solution was centrifuged at 10,000 g for 10 min at 4 °C. The pellet obtained was suspended in 45 mL sodium phosphate buffer (25 mM, pH 7) containing 1.3 M (NH₄)₂SO₄. The enzyme solution was filtered (0.45 µm) for the subsequent HIC purification using the resin Toyopearl Phenyl-650M (Column volume (CV) = 22 mL). The sample was applied to the column at a flow rate of 2 mL/min. The column was washed with 4 CV of binding buffer (25 mM sodium phosphate buffer + 1.3 M (NH₄)₂SO₄; pH 7.0) at 3 mL/min. The DAO-1 was eluted in a linear gradient of increasing (0 to 100 % (v/v)) elution buffer (25 mM sodium phosphate buffer; pH 7.0) over 9 CV with a subsequent gradient delay of 50 mL. Samples taken during each purification step were desalted using PD-10 columns (GE Healthcare, Chicago, USA) against PIPES buffer (25 mM, pH 7.2), according to the manufacturer's instructions.

### Protein analysis

The protein content of enzyme samples was determined according to Bradford, using BSA as a standard, as known in the art. Samples of the DAO purification procedure were analyzed by sodium dodecyl sulfate (SDS)-polyacrylamide gel electrophoresis (PAGE) on a 10% separating gel, as known in the art. An amount of 5 µg protein was loaded onto each lane of the SDS-PAGE. A protein molecular mass standard was used (Precision Plus Protein^{™} unstained protein standard 10 - 250 kDa) for molecular mass determination. Coomassie Brilliant Blue G-250 was used to stain the gel, as known in the art. The purified DAO-1 was additionally investigated by native PAGE analysis. The purified DAO-1 was applied twice on the native PAGE (each 5 µg protein), whereby one part was stained with Coomassie Brilliant Blue G-250 and the other part was active stained. The active staining solution contained 30 mM histamine, 50 µM DA-67 reagent, 5.32 U/mL horseradish peroxidase (Grade I) and 25 mM PIPES (pH 7.2). The active staining was continued at 37 °C until a blue band became visible.

### DAO activity determination

The DAO activity was determined using the colorimetric DA-67 enzyme assay, as known in the art. The reaction mixture, containing 750 µL histamine solution (30 mM; dissolved in 25 mM PIPES; pH 7.2; flushed with O₂ for 5 min) and 726 µL DA-67 reagent (10-(carboxymethyl-aminocarbonyl)-3,7-bis(dimethylamino) phenothiazine sodium salt; 50 µM; dissolved in 25 mM PIPES; pH 7.2) was incubated at 37 °C for 10 min and stirred at 750 rpm. Subsequently, 24 µL (266 units/mL) of horseradish peroxidase (Grade I) were added. The reaction was started by the addition of 50 µL enzyme solution and incubated at 37 °C for 10 min and stirred at 750 rpm. The reaction was stopped by the addition of 50 µL sodium diethyldithiocarbamate (30 mM). After centrifugation (10,000 g for 3 min at 20 °C), the absorption was measured at 668 nm. The histamine solution was replaced with buffer (25 mM PIPES; pH 7.2) for reference. Hydrogen peroxide (0.5 to 10 nmol/mL) was used for the calibration. The enzyme activity was calculated in nkat, whereby 1 nkat converts 1 nmol substrate/s at 37 °C.

### Investigation of the temperature and pH profile of DAO-1

The influence of temperature on the enzyme activity of DAO-1 was tested under standard assay conditions, whereby the incubation temperature was varied between 20 and 47 °C. The pH-dependency of the DAO-1 activity was investigated under standard assay conditions at 37 °C, whereby different buffer systems (50 mM sodium phosphate buffer, pH 6.2 - 7.8; 50 mM PIPES, pH 7 - 7.5; 50 mM Tris-HCl, pH 7.2 - 8.5) were used. The respective buffer system was calibrated with hydrogen peroxide (0.5 to 10 nmol/mL). Analytically pure histamine was used as a substrate for the temperature and pH profiles.

### Investigation of the pH-stability of DAO-1

The influence of buffer and pH value on the stability of DAO-1 was investigated to determine suitable conditions for the histamine bioconversion experiment. Consequently, DAO-1 was incubated for 5 h at 37 °C and stirred at 400 rpm in the following buffer systems: 20 mM MES (pH 5.5), 20 mM sodium phosphate buffer (pH 6.2, 7.0, 7.2, 7.8, 8.0), 20 mM PIPES (pH 7.0, 7.2, 7.5) and 20 mM Tris-HCl (pH 6.8, 7.5, 8.2). Afterwards, the residual enzyme activity of DAO-1 was determined under standard assay conditions and compared to the DAO-1 activity applied initially.

### Kinetic characterization of DAO-1

The apparent kinetic parameters of DAO-1 were determined by Michaelis-Menten kinetics with histamine as the substrate under standard assay conditions. The histamine concentration (analytically pure histamine) was varied between 0.5 and 50 mM. Kinetic investigations were done within the initial reaction velocity.

### Investigation of the substrate selectivity of DAO-1

The substrate selectivity of DAO-1 was tested with different food industry-relevant biogenic amines as substrates (tryptamine, cadaverine, putrescine, agmatine, spermidine, histamine and tyramine) under standard assay conditions. The amino acids L-tryptophan, L-lysine, L-histidine and L-arginine were also tested. Each substrate was used at concentrations of 1, 10 and 50 mM in the final assay approach, except tryptamine, which was tested only at 1 mM due to its insolubility at higher concentrations.

### Histamine bioconversion with DAO-1

The histamine reduction experiments were done, as known in the art, with a food-relevant histamine concentration of 150 mg/L histamine. The histamine reduction was done at 37 °C in PIPES buffer (20 mM, pH 7) for 5 h. An amount of 5 g/L BSA and 25 g/L sucrose were added to the approach to simulate a food matrix. Additionally, catalase was added (30 nkat/mL) to remove the hydrogen peroxide. The experiment was started by the addition of 0.1 nkat/mL DAO-1 activity. This DAO activity was determined under modified assay conditions, whereby the buffer system (20 mM PIPES, pH 7), substrate concentration (1.35 mM histamine in the final assay approach) and additives (5 g/L BSA and 25 g/L sucrose) were adjusted to the bioconversion conditions. The histamine reductions were determined by reversed-phase high-performance liquid chromatography with thiamine (1.35 mM) as an internal standard, as known in the art.

### Statistical analysis

All experiments, except the DAO-1 production in the bioreactor, were carried out at least in duplicate and evaluated by determining the standard deviation with Excel (Microsoft, Redmond, USA). Data are presented as mean values with standard deviation. Enzyme kinetics were evaluated by nonlinear regression using the data analyzing software Sigmaplot 12.5 (Systat Software GmbH, Erkrath, Germany).

### Example 1:

### Identification of dao genes in Y. lipolytica PO1f and their expression in shaking flask experiments

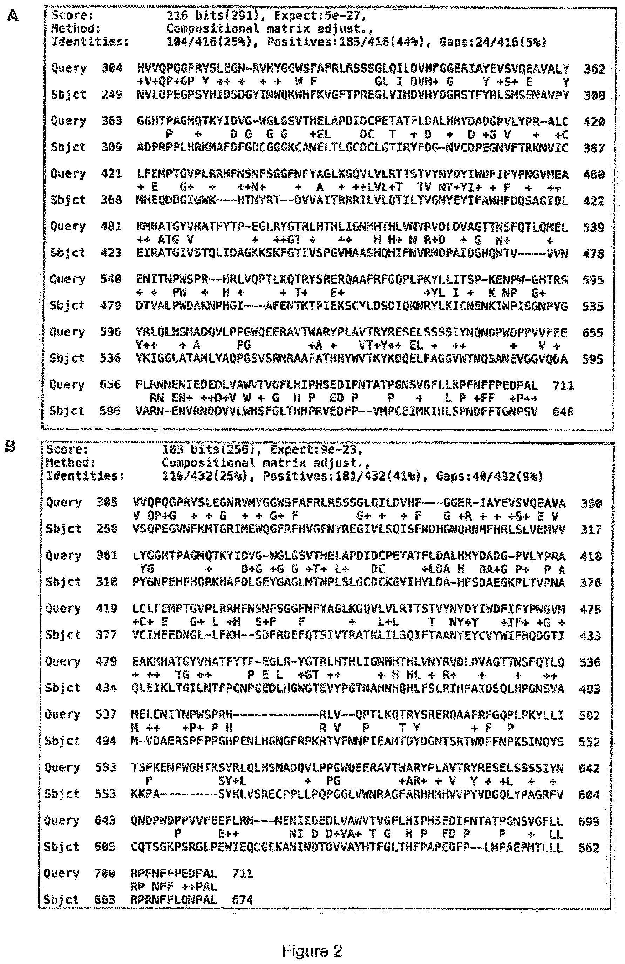

*Y. lipolytica* PO1f was identified as the owner of two putative DAOs using the BLAST program (https://blast.ncbi.nlm.nih.gov). An amino acid sequence comparison of porcine DAO with the two putative DAOs showed a query cover of 54 % and an identity of 25 % for DAO-1 and a query cover of 53 % and an identity of 25 % for DAO-2 (Fig. 2A, B). Comparing both putative DAOs with each other showed a query cover of 97 % and an identity of 35 %. Both genes were amplified from the genome of the *Y. lipolytica* strain PO1f and successfully integrated into the *axp* locus of the genome (integration efficiency of around 20 %), resulting in the recombinant strains *Y. lipolytica* PO1f*_axp_dao-1* and *Y*. *lipolytica* PO1f*_axp_dao-2.* The genes were integrated into the *axp* locus because it allowed efficient expression of the proteins desired with the chosen strategy for homologous recombinant integration, as known in the art. *Y*. *lipolytica* PO1f_*axp_dao-1* and *Y. lipolytica* PO1f*_axp_dao-2* were cultivated in shaking flasks to screen for the clones with the highest DAO activity. However, the putative DAO-2 showed no DAO activity towards histamine and only weak activity towards agmatine (around 50 nkat_{Agmatine (14.5 mM)}/L_{Culture}). Therefore, only DAO-1 was investigated further in the present invention.

### Example 2:

### Production of DAO-1 in Y. lipolytica PO1f

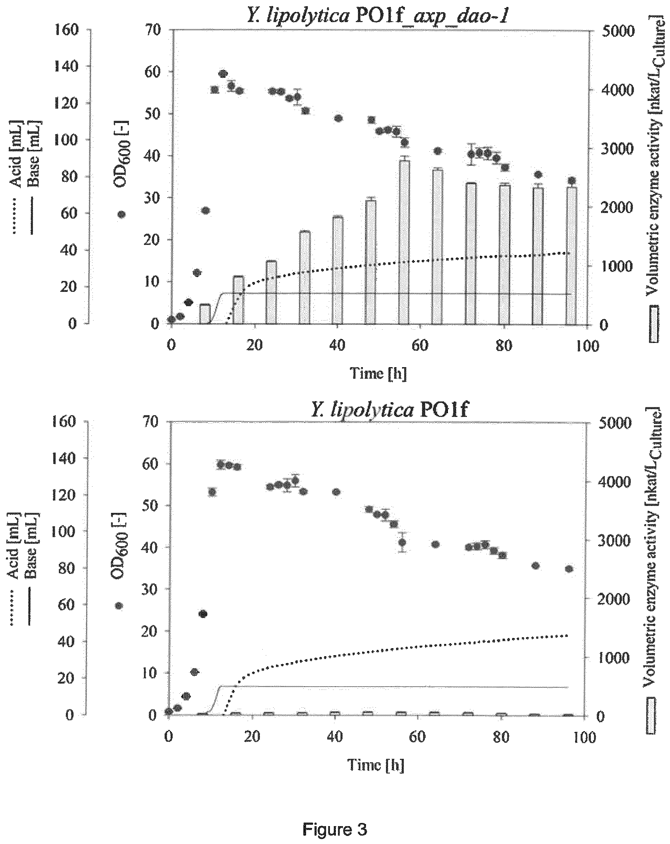

*Y. lipolytica* PO1f_*axp_dao-1* was cultivated in the Multifors bioreactor system in working volumes of 800 mL in YPD medium supplemented with 0.2 g/L uracil over 96 h. The original *Y. lipolytica* PO1f strain was cultivated identically in parallel as a reference. Both strains showed similar growth behaviors reaching the maximal OD₆₀₀ of around 60 after 12 h (maximal specific growth rates *µ* of 0.32 h⁻¹), as shown in Figure 3. The OD₆₀₀ of both strains decreased to around 34, which corresponded to a bio dry mass of 15 g/L after 96 h of cultivation. The volumetric DAO-1 activity (intracellular) of the *Y. lipolytica* PO1f_*axp_dao-1* strain increased linearly over the course of the bioreactor cultivation, reaching a maximum of 2784 ± 75 nkat/L_{Culture} after 56 h. Then, the DAO-1 activity decreased slightly, reaching a final volumetric DAO-1 activity of 2343 ± 98 nkat/L_{Culture} with a specific DAO-1 activity of 1301 ± 54.2 nkat/g_{Protein}. The reference *Y. lipolytica* PO1f had a maximal volumetric DAO activity of 58 ± 0.6 nkat/L_{Culture} with a specific DAO activity of 14.1 ± 0.14 nkat/g_{Protein} after 48 h of cultivation. It is suggested that the DAO activity of the reference strain *Y. lipolytica* PO1f is due to the native DAO-1 expression, since DAO-2 showed no activity towards the substrate histamine, which was used for the determination of DAO activity. Therefore, the homologous recombinant integration of the *dao-1* gene into the *axp* locus of *Y. lipolytica* PO1f allowed efficient expression of DAO-1.

The specific DAO-1 activity of 1301 ± 54.2 nkat/g_{Protein} obtained in the crude extract was around 16 times higher than the DAO activity of the partially purified porcine DAO known in the art with 81 nkat/g_{Protein}. If DAO is used as a dietary supplement, a considerably high activity of DAO must be used to degrade the food-relevant histamine amounts. The enzyme activity of porcine DAO required for the degradation of 75 mg histamine was 50 nkat within 5 h under *in vitro* conditions. To obtain this DAO activity, 100 g of pig kidney were needed for sufficient DAO extraction. By comparison, the isolation and partial purification of DAO from almost 5 kg pig kidneys would result in the same amount of DAO-1 activity obtained from one liter of bioreactor cultivation of *Y. lipolytica* PO1f_*axp_dao-1.* Therefore, the microbial DAO production seems cost-effective and offers several advantages compared to the use of DAO from pig kidney, especially in terms of ethical and cultural considerations.

### Example 3:

### Purification of DAO-1 from crude extract after cell disruption

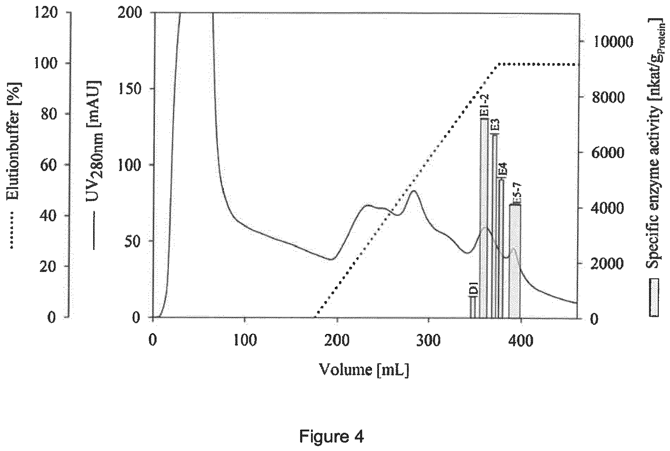

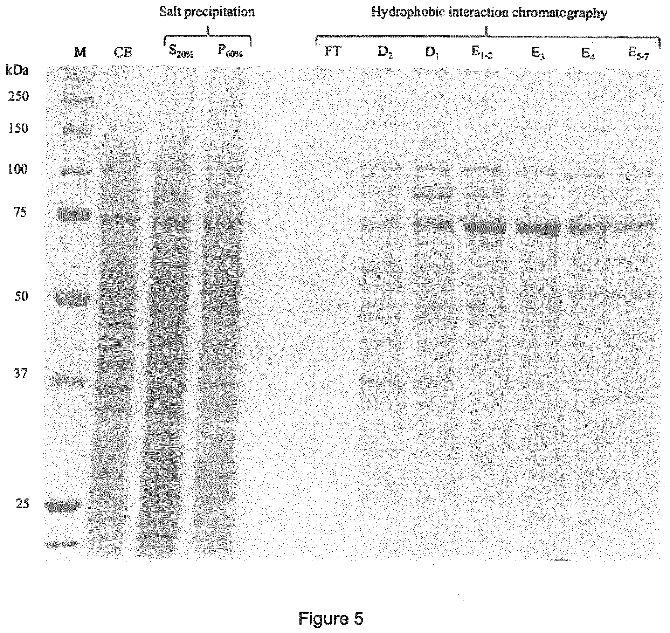

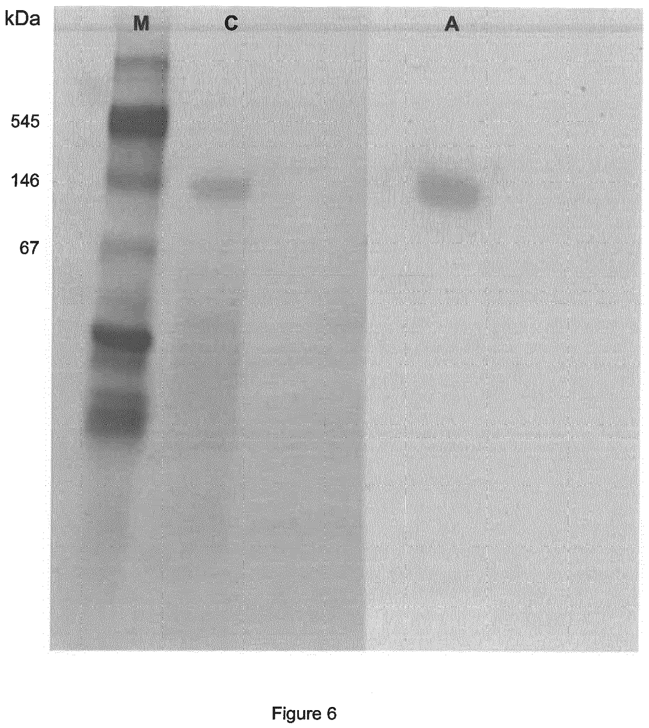

The yeast DAO-1 was purified by fractionated (NH₄)₂SO₄ precipitation and subsequent HIC. From 10 g of wet yeast biomass, 108 ± 5 nkat of DAO-1 activity (specific DAO-1 activity of 660 ± 28 nkat/g_{Protein}) was obtained after cell disruption using a high-pressure homogenizer. After (NH₄)₂SO₄ precipitation and HIC (Fig. 4), the DAO-1 was purified 7.2-fold (specific enzyme activity of 4738 ± 31 nkat/g_{Protein}) with a yield of 46.6 ± 0.31 %. By comparison, the purification of DAO from porcine kidney yielded a specific enzyme activity of 81 ± 1 nkat/g_{Protein} with a similar purification strategy employing a heat treatment, salt precipitation and HIC. Thus, the production of a DAO in a microbial production host and the subsequent purification achieved much higher specific DAO activities. The purification of DAO-1 was evaluated by SDS-PAGE analysis (Fig. 5) showing a protein band with increasing intensity over the purification procedure at around 75 kDa, which was in accordance with the theoretical monomeric molecular weight of DAO-1 (75.4 kDa as calculated from amino acid sequence). The SDS-PAGE also showed that the DAO-1 preparation still contained other proteins. However, it was shown on an active-stained native PAGE (Fig. 6) that only one protein band at around 146 kDa was active towards the substrate histamine. This indicated that the purity of the DAO-1 preparation was sufficient for further biochemical characterization. The DAO-1 seemed to be a homodimeric enzyme based on the size observed in SDS-PAGE and native PAGE analysis, respectively. This was in accordance with the homodimeric structure observed for most of the copper amine oxidases including human and porcine DAO described in the art.

### Example 4:

### Biochemical investigation of DAO-1

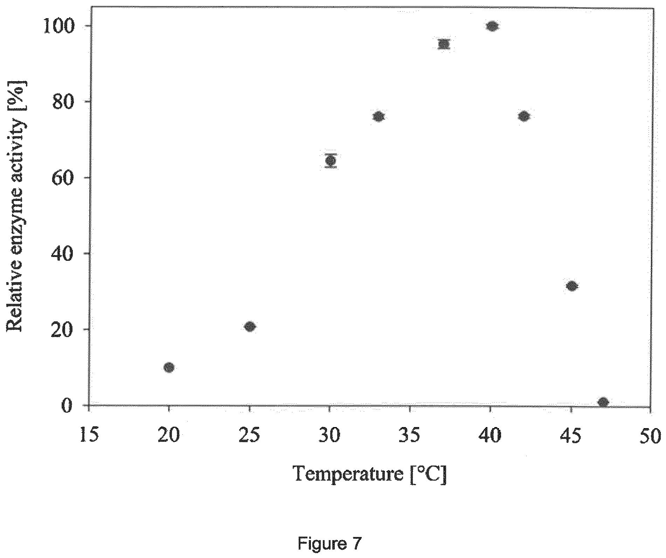

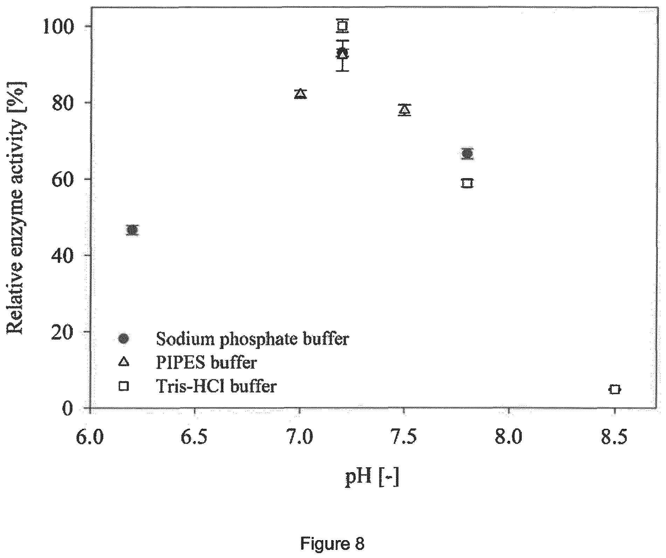

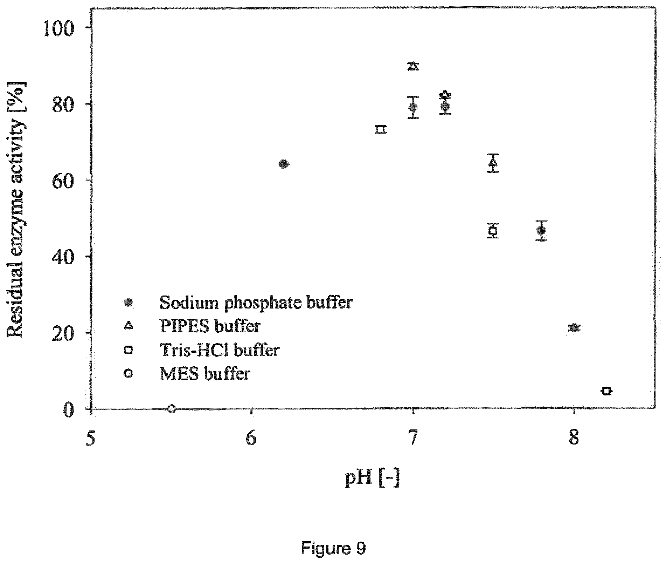

The purified DAO-1 was investigated regarding the influence of temperature, pH value and buffer on the DAO-1 activity. The DAO-1 had the maximal activity at 40 °C (Fig. 7). An increase of the temperature to 42 or 45 °C decreased the relative DAO-1 activity to 76 ± 1% and 31.7 ± 1 %, respectively. The relative DAO-1 activity was greater than 50 % for a temperature range between 30 and 42 °C. Therefore, the temperature profile of DAO-1 was comparable to the temperature profiles of other DAOs, for example, that from pig and human, however, these DAO activities were determined with putrescine as the substrate. The DAO-1 showed the maximal DAO activity at pH 7.2 in Tris-HCl buffer (50 mM) (Fig. 8). Due to the limited buffering capacity of Tris-HCl at this pH value, the following experiments were done in PIPES buffer, which resulted in a slightly lower DAO-1 activity of 92.2 ± 3.9 % compared to the maximal activity in Tris-HCl at pH 7.2. The pH-profile also seems to be comparable to human and porcine DAOs. The prior art found that the maximal DAO activity of human DAO (from blood serum) with putrescine as the substrate was at pH 7.5 in phosphate buffer. The DAO from pig kidney showed the maximal DAO activity towards histamine and cadaverine in potassium phosphate buffer at pH values of 6.3 and 7.4, respectively. The highest stability of DAO-1 was determined in PIPES buffer (20 mM) at pH 7.0 with a residual DAO-1 activity of 89.5 ± 0.8 % after incubation at 37 °C for 5 h (Fig. 9). The DAO-1 still retained 53.7 ± 1.2% of its activity (data not shown) after 24 h at 37 °C. The DAO-1 maintained more than 60 % of 390 its activity after a 5 h incubation at 37 °C in a pH range from 6.2 to 7.5.

### Example 5:

### Kinetic investigation of DAO-1

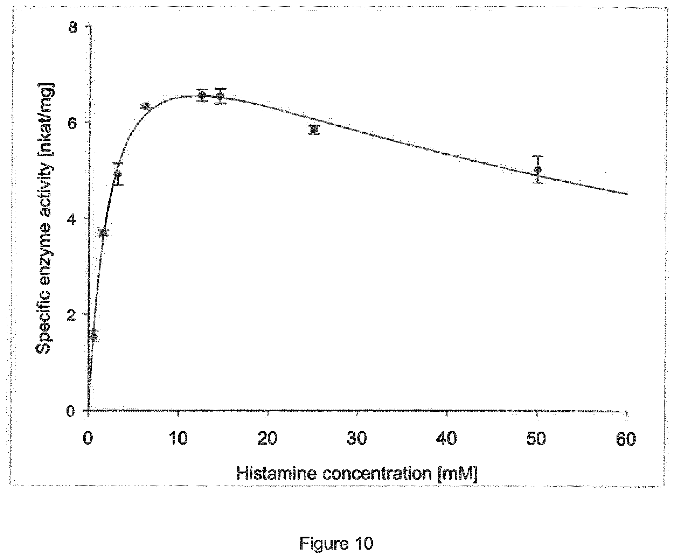

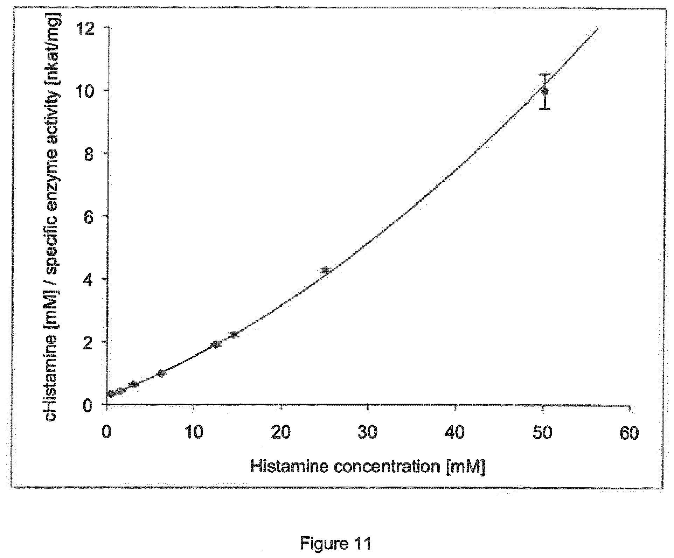

The kinetic investigation of DAO-1 was carried out with histamine concentrations ranging from 0.5 to 50 mM (Fig. 10). The linearization of the Michaelis-Menten curve, according to Hanes-Woolf, is shown in Figure 11. A substrate inhibition by histamine was recognized for histamine concentrations greater than 12.5 mM. The *K*ₘ value of DAO-1 for histamine was 2.3 ± 0.2 mM (R² = 0.985) with a *V*ₘₐₓ of 9.09 ± 0.37 nkat/mg. Furthermore, a *K*ᵢ of 61.89 ± 8.51 mM was determined. Therefore, the affinity of DAO-1 to the substrate histamine is less compared to mammalian DAOs from porcine or human, which have *K*ₘ values for histamine of 0.027 and 0.0028 mM, respectively. However, microbial amine oxidases seem to generally have less affinity towards histamine compared to mammalian DAOs. *K*ₘ values of 0.5 and 0.6 mM were determined in the art for a histamine oxidase from *Arthrobacter crystallopoietes* KAIT-B-007 and an amine oxidase from *Aspergillus niger,* respectively, with histamine as substrate. The comparatively lower affinity of microbial DAOs towards histamine might derive from the fact that mammalian DAOs are detoxifying enzymes that must be able to degrade biogenic amines at very low concentrations with high efficiency. However, the DAO in microorganisms seems to be primarily important for the nitrogen metabolism and, therefore, does not need to have a very high affinity towards histamine.

The porcine and human DAOs are also substrate inhibited by histamine but at distinctively lower concentrations of *K*ᵢ = 5.71 and 0.28 mM, respectively. Therefore, DAO-1 degrades histamine at higher concentrations with a higher reaction rate.

### Example 6:

### Investigation of the substrate selectivity of DAO-1

The DAO-1 showed a broad substrate selectivity with different food-relevant biogenic amines as substrates (Tab. 1). The highest DAO-1 activity was determined with 1 mM tyramine as the substrate. The DAO-1 seemed to be substrate-inhibited by tyramine, as an increase of the substrate concentration caused a reduction of the DAO-1 activity. The same inhibitory effect was also observed for the other substrates: Histamine, putrescine, cadaverine, agmatine and spermidine. The third highest DAO-1 activity was measured with histamine as the substrate at 10 mM, which was set as a reference (100 %) since it was the biogenic amine with the highest relevance for the present invention. The DAO-1 did not show any activity towards the amino acids L-tryptophan, L-lysine, L-histidine and L-arginine tested.

**Table 1. Substrate selectivity of DAO-1 towards food-relevant biogenic amines at 1, 10 and 50 mM. DAO-1 activity measured with the DA-67 assay in PIPES buffer (25 mM; pH 7.2). 100 % (10 mM histamine) = 1.45 ± 0.01 nkat/mL.**

| Substrate | DAO-1 activity [%] | | |
|---|---|---|---|
| | 1 mM | 10 mM | 50 mM |
| **Histamine** | **45.1 ± 0.7** | **100 ± 1.5** | **74.4 ± 1.1** |
| Tyramine | 303.9 ± 4.4 | 246.8 ± 3.6 | 66.4 ± 1.0 |
| Putrescine | 29.8 ± 0.4 | 92.9 ± 1.4 | 77.9 ± 1.1 |
| Cadaverine | 29.6 ± 0.4 | 87.3 ± 1.3 | 64.5 ± 1.0 |
| Agmatine | 31 ± 1.1 | 30.6 ± 2.7 | 7.4 ± 2.3 |
| Spermidine | 2.1 ± 1.6 | 3.8 ± 0.9 | 2.1 ± 1.3 |
| Tryptamine | 166.1 ± 0.8 | not soluble | not soluble |

| | | | |
|---|---|---|---|
| The amino acids L-tryptophan, L-lysine, L-histidine and L-arginine were tested but did not show DAO-1 activity. | | | |

The biogenic amines tyramine, histamine, putrescine and cadaverine are especially of high relevance when it comes to biogenic amine-induced toxicity from foods. The DAO-1 was capable of degrading all of these relevant biogenic amines with high efficiency. This broad substrate selectivity for food-relevant biogenic amines seems to be a rarely found feature of microbial amine oxidases. The histamine oxidase from *Arthrobacter crystallopoietes* KAIT-B-007 and an amine oxidase from *Aspergillus niger,* for example, showed no activity towards the substrates putrescine, cadaverine, spermine and spermidine but showed activity towards histamine. A phenylethylamine oxidase from *Arthrobacter globiformis* was also found in the art to be active towards tyramine but was poorly active towards histamine and not active towards putrescine.

The mammalian DAO from porcine kidney showed the highest activity towards the aliphatic diamines putrescine and cadaverine in the art, both at 1 mM. Here, the DAO activity measured with histamine was 19 % compared to the maximal measured activity with putrescine.

### Example 7:

### Histamine bioconversion with DAO-1

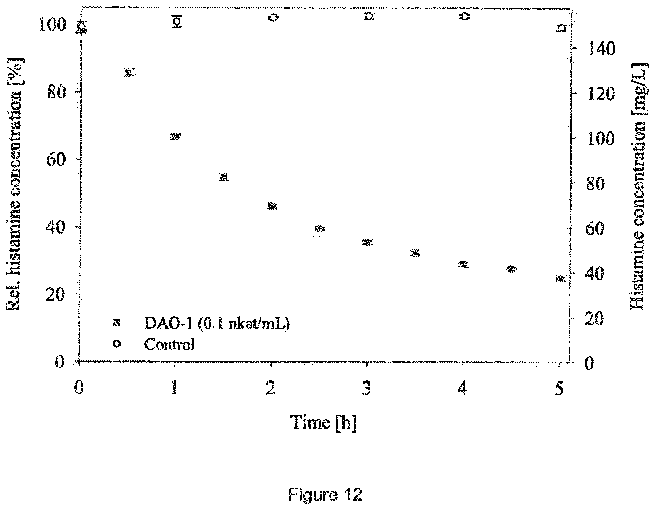

The DAO-1 was used in histamine bioconversion experiments to evaluate its efficacy for the reduction of histamine in food-relevant concentrations (150 mg/L; 1.35 mM), as known in the art, with modifications regarding the buffer system (20 mM PIPES; pH 7) and used additives to simulate a food matrix (5 g/L BSA, 25 g/L sucrose, 30 nkat/mL catalase) (Fig. 12). The DAO-1 preparation reduced the histamine by 74.9 ± 1.73 % to 37.4 ± 0.65 mg/L (0.34 ± 0.01 mM) using 0.1 nkat/mL within 5 h at 37 °C. Although the histamine bioconversion experiments were carried out at histamine concentrations slightly above the *K*ₘ value of DAO-1 (2.3 ± 0.2 mM), histamine was steadily reduced until a stagnation was recognized, starting at around 4 h at a histamine concentration of 43.5 ± 0.76 mg/L (0.39 ± 0.01 mM). The residual activity of DAO-1 after this 5 h bioconversion was 57.4 ± 1.3 % (determined by RP-HPLC). Therefore, the stagnation was attributed to the enzyme kinetics of DAO-1, which decelerated the enzyme catalysis at lowered histamine concentrations. Nevertheless, the DAO-1 kinetics were sufficient for the conversion of around 75 % of the histamine applied. Around 90 % of the histamine applied (150 mg/L) were reduced using the same enzyme activity as used herein in previous studies with porcine DAO. Therefore, porcine DAO was also unable to completely degrade the histamine in the experimental setup, which was attributed to a potentially product inhibitory effect by (imidazol-4-yl)acetaldehyde. However, it was also stated that this effect is difficult to assess for the histamine conversion in actual foods, because the reaction product might react further to other compounds and, therefore, would not disturb the actual histamine conversion.

### Discussion:

*Y. lipolytica* PO1f was identified as the producer of a DAO (DAO-1) which showed a broad substrate selectivity, including the most relevant biogenic amines histamine, putrescine, cadaverine, and tyramine. This seems to be a rarely found feature in microbial DAOs, which makes DAO-1 an interesting enzyme for administration in the food industry. The DAO-1 showed similar biochemical characteristics regarding the temperature and pH profile compared to porcine and human DAOs. As observed for other microbial DAOs, DAO-1 showed a lower affinity (Km = 2.3 ± 0.2 mM) towards the substrate histamine compared to the mammalian porcine and human DAOs. Nevertheless, DAO-1 was capable of reducing around 75 % of the histamine applied (150 mg/L) in a histamine bioconversion experiment. The cost-effective and convenient production of DAO-1 in *Y. lipolytica* PO1f and its biochemical characteristics makes it an interesting enzyme for application in the food industry for the degradation of biogenic amines such as histamine, as well as for a range of medical applications in the prevention and treatment of conditions and diseases associated with the ingestion of biogenic amines.

Specifically, in the present invention, a putative diamine oxidase (DAO) from *Yarrowia lipolytica* PO1f (DAO-1) was homologously recombinantly integrated into the genome of *Y. lipolytica* PO1f using the CRISPR-Cas9 system for the subsequent DAO production in a bioreactor. Thereby, it was proven that the DAO-1 produced was indeed a functional DAO. The cultivation yielded 2343 ± 98 nkat/L culture with a specific DAO activity of 1301 ± 54.2 nkat/g_{Protein}, which was a 93-fold increase of specific DAO activity compared to the native *Y. lipolytica* PO1f DAO-1 production. The DAO-1 was most active at 40 °C, pH 7.2 in Tris-HCl buffer (50 mM) (with histamine as substrate), which is comparable to human and porcine DAOs. With its broad selectivity for the most relevant biogenic amines in foods, DAO-1 from *Y. lipolytica* PO1f is an interesting enzyme for application in the food industry for the degradation of biogenic amines, as well as for a range of medical applications in the prevention and treatment of conditions and diseases associated with the ingestion of biogenic amines.

The present invention relates to the following amino acid and nucleotide sequences:
SEQ ID NO: 1
   Amino acid sequence of DAO-1 from *Y. lipolytica* PO1f 
MTPHPFDQLSVQEMESVVRVVKSNHSGKSLHLKSIGTEEPPKALMAPFLAAKRAGKNPV
PPPRIAHVIYYVLEDKLVNQCWVDVPSAKVVKSEVLKKGIHPPIDPWEANEAFEAAFDH
PLVKDAIKKCGVEHLIDNLTIDGWMYGCDSEIDMPRYLQMLVYCRDPKTNHQDSNMYAF
PVPFVPVYDVLEKKLVRVDYCATGGDDDAAVEGVGNYDTRPEGKNCIEHCVTNDYLPE
LQDKMRTDLKPYNVLQPEGPSYHIDSDGYINWQKWHFKVGFTPREGLVIHDVHYDGRST
FYRLSMSEMAVPYADPRPPLHRKMAFDFGDCGGGKCANELTLGCDCLGTIRYFDGNVCD
PEGNVFTRKNVICMHEQDDGIGWKHTNYRTDVVAITRRRILVLQTILTVGNYEYIFAWH
FDQSAGIQLEIRATGIVSTQLIDAGKKSKFGTIVSPGVMAASHQHIFNVRMDPAIDGHQ
NTVVVNDTVALPWDAKNPHGIAFENTKTPIEKSCYLDSDIQKNRYLKICNENKINPISG
NPVGYKIGGLATAMLYAQPGSVSRNRAAFATHHYWVTKYKDQELFAGGVWTNQSANEVG
GVQDAVARNENVRNDDVVLWHSFGLTHHPRVEDFPVMPCEIMKIHLSPNDFFTGNPSVD
VPKSNQTFNRSVEVKDCRSCKI
SEQ ID NO: 2
   Gene *dao-1* from *Y. lipolytica* PO1f 
atgactccccaccctttcgatcagctctccgttcaggagatggagagcgttgtgcgagt
ggtcaagtccaaccattcgggcaagtctcttcacctcaagtccattggcaccgaggagc
ctcccaaggcgctgatggctcctttccttgcagccaagcgAgctggcaagaaccccgtt
cccccccctcgaatcgcacatgtcatctactatgttctggaggacaagttggtgaacca
gtgctgggtcgatgttccttccgccaaggttgtcaagtccgaggtgctcaagaagggca
tccatcctcccattgatccctgggaggccaacgaggccttcgaggccgcctttgaccat
cctctggtcaaggacgccatcaagaagtgcggcgtggagcatctgatcgacaacctcac
aattgacggctggatgtacggctgtgacagcgagattgacatgccccggtacctgcaga
tgctggtctactgccgagatcccaagaccaaccaccaggactccaacatgtacgccttc
cccgttccgtttgttcctgtctacgacgtgctggagaagaagctcgttcgagtcgacta
ttgcgccaccggtggagacgatgacgacgctgccgtcgagggcgttggcaactacgaca
cccgacccgagggaaagaactgcatcgagcactgtgtcaccaacgactatcttcccgag
 
cttcaggacaagatgcgaaccgacctcaagccctacaacgtgttgcagcccgagggtcc
ctcttaccacattgacagtgacggctacatcaactggcaaaagtggcacttcaaggtcg
gattcactccccgagagggtctggtgatccacgatgtccactacgacggccgatccacc
ttctaccgactgtccatgtccgagatggcggttccctacgccgatccccggcccctct
gcaccgaaagatggcgtttgatttcggcgactgtggaggAggaaagtgcgccaacgagc
tgactctgggctgcgactgtcttggtaccatccgatactttgacggcaacgtgtgcgac
cccgagggcaacgtgttcacccgaaagaacgtcatctgtatgcacgagcaggatgacgg
tatcggctggaagcacactaactaccgaaccgacgtggttgccatcacccgacgacgaa
ttctggttctgcagaccattctgaccgtgggcaactacgagtacatctttgcctggcac
tttgaccagtctgccggaatccagctggagatccgagccaccggaatcgtctccaccca
gcttatcgacgccggcaagaagtccaagtttggcaccattgtctctcccggagtcatgg
ccgcctctcaccagcacattttcaacgtgcgaatggaccctgccattgacggccatcag
aacacagttgtggtcaacgacaccgttgctctgccctgggacgccaagaacccccatgg
aatcgcctttgagaacaccaagacccccatcgagaagtcgtgctacctggactcggaca
tccagaagaaccggtacctcaagatctgcaacgagaacaagatcaaccccatctccggc
aaccccgtgggCtacaagattggaggtctggccaccgccatgctgtacgctcagcccgg
ctccgtttcgcgaaaccgagccgcctttgccacccaccactactgggtcaccaagtaca
aggaccaggagctgtttgccggCggcgtgtggaccaaccagtctgccaacgaggtcggc
ggagtccaggatgccgttgcccgaaacgagaacgtgcgaaacgacgacgtggttctctg
gcactcctttggtctcactcaccacccccgagtcgaggacttccccgtcatgccttgcg
agattatgaagatccatctgtcgcccaacgatttcttcaccggcaacccttctgttgac
gtgcccaagtccaaccagactttcaatcgatccgtcgaggtcaaggactgtcgatcttg
caagatctag
 Nucleotides in underlined, bold capital letters indicate silent mutations with respect to the putative DNA sequence given in the NCBI database.
SEQ ID NO: 3
   Gene *dao-2* from *Y. lipolytica* PO1f 
atgcacagactatcacaactagctacacaaacaaccgcggccaccatcaccgcaggtca
tcctcttgatcctctctctccctccgaaatcgaacatgccgcttccatcgtcaaatcgc
agatgcgagactcgtctccgtaccggttcaatctcatcacgctgatcgagccgcccaag
gccgagcttcttgcgtgggaggcgtcgccttcctcggtggccaaacctcctcgacgagc
 
ggaggtcgtactcgttgtgagaggcaagaagggcgtcaccgagggccgggtctgtctca
ccggctcaaaggtgctttcgtggtccgaaattgaggcgtccagcctattcttaccgtt
gacgacctccaaaaggtcgaggaaattgtgcgacaggaccccgaagtcatcaagcagtg
caaactcattggagttgacaacatgtcccaggtgtactgtgatccctggactattggct
atgacgaaagatggggtgccgaacgacgtctacagcaggcgttcctctacttccgagcc
caccaggacgactcccagtactcccatcctcttgacttcactccaatctacgacgccac
ggagcagaaagtcatctttattgatattcctcccgttcgtcgacctctctccaagctta
agaattccaacttcaaccctcaggatatctccaagactaccggttacagagacgtgaag
cccatcgacgtgtctcagcccgaaggagtcaacttcaagatgaccggtcgaatcatgga
gtggcagggattccgatttcacgtgggattcaactacagagagggaattgtgctgtctc
agatctctttcaacgaccatggtaaccagcgaaacatgttccatcgtctctctctcgtt
gaaatggttgttccctacggaaaccccgagcaccctcaccagcgaaagcacgcctttga
tctgggagagtacggagccggtctcatgaccaatcctctttccctcggatgtgattgca
agggagtcattcactaccttgatgcgcacttttccgacgccgaaggaaagcctctcact
gttcccaacgctgtgtgtatccacgaggaggacaatggtctgcttttcaagcactctga
cttccgagacgagttccagacttcgatcgtcactcgagctaccaagctcatttgtcgc
agattttcaccgccgcaaactacgaatactgcgtctactggattttccaccaggacggt
accatccagctggagattaagctcaccggcatcctcaacactttcccctgcaatcccgg
agaagatttgcatggctggggcacagaggtgtaccctggtaccaacgcccacaaccatc
agcatctcttttctctgcgaatccatcctgccattgattcccagctgcatccaggtaat
tctgtggcaatggtggacgctgagcggtcacccttcccgcctggacacccagaaaacct
gcacggcaatggtttccggcccaagcgaacggtcttcaacaacccgatcgaggctatga
cggattatgatggtaatacatcgcgaacttgggactttttcaacccgaagtccatcaac
cagtactccaagaagcccgcttcttacaagctggtgtctcgtgagtgccctcctctgct
tcctcagcctggtggactggtttggaaccgagctggttttgcacgacaccatatgcatg
ttgttccgtatgtggacggccagctgtaccctgctggacggtttgtttgccagacaagt
ggaaagccctcaaggggtctccccgagtggattgagcagtgcggagagaaggccaatat
caacgataccgatgttgttgcctatcacacttttggtctgacccatttccctgctcccg
aggacttccctttgatgcctgccgagcccatgactctactttgcgacccagaaacttc
ttcctgcagaacccggctttggacgtgcctccttcgcatgctcgaaccaccacagaggc
ccaggctgcttccggtgctaaggttgtctctcttaccgacaaggtgtcgcagctggctt
tcaataagtcgtgctgttccaagtag
SEQ ID NO: 4
   Forward primer for the amplification of dao-1 from the genomic DNA of Y. *lipolytica* PO1f
   atgactccccaccctttcgatcag
SEQ ID NO: 5
   Reverse primer for the amplification of dao-1 from the genomic DNA of Y. *lipolytica* PO1f
   ctagatcttgcaagatcgacagtccttg
SEQ ID NO: 6
   Forward primer for the second amplification of *dao-1* and addition of restriction sites
   cttgcgcgcatgactccccaccctttc
SEQ ID NO: 7
   Reverse primer for the second amplification of *dao-1* and addition of restriction sites
   ggcgctagcctagatcttgcaagatcg
SEQ ID NO: 8
   Forward primer for the amplification of *dao-2* from the genomic DNA of *Y*. *lipolytica* PO1f and addition of restriction sites
   gaagcgcgcatgcacagactatcacaactagc
SEQ ID NO: 9
   Reverse primer for the amplification of dao-2 from the genomic DNA of *Y.* *lipolytica* PO1f and addition of restriction sites
   caagctagcctacttggaacagcacga
SEQ ID NO: 10
   Forward sequencing primer for *dao* genes
   ctaaagatgttgatctccttgtgcc
SEQ ID NO: 11
   Reverse sequencing primer for *dao* genes
   cctctgggccgaatacaacac

## Claims

1. An enzyme having diamine oxidase activity for use in medicine, wherein said enzyme comprises
(i) the amino acid sequence of SEQ ID NO: 1; or
(ii) an amino acid sequence having at least 70% sequence identity to SEQ ID NO: 1 and having diamine oxidase activity.

2. The enzyme for use according to claim 1, wherein the enzyme consists of
(i) the amino acid sequence of SEQ ID NO: 1; or
(ii) the amino acid sequence having at least 70% sequence identity to SEQ ID NO: 1 and having diamine oxidase activity.

3. The enzyme for use according to claim 1 or claim 2, wherein the amino acid sequence in (ii) has at least 72%, at least 74%, at least 76%, at least 78%, at least 80%, at least 82%, at least 84%, at least 86%, at least 88%, at least 90%, at least 91%, at least 92%, at least 93%, at least 94%, at least 95%, at least 95.5%, at least 96%, at least 96.5%, at least 97%, at least 97.5%, at least 98%, at least 98.2%, at least 98.4%, at least 98.6%, at least 98.8%, at least 99%, at least 99.1%, at least 99.2%, at least 99.3%, at least 99.4%, at least 99.5%, at least 99.55%, at least 99.6%, at least 99.65%, at least 99.7%, at least 99.75%, at least 99.8%, at least 99.85%, at least 99.9%, at least 99.91%, at least 99.92%, at least 99.93%, at least 99.94%, at least 99.95%, at least 99.96%, at least 99.97%, at least 99.98%, or at least 99.9% sequence identity to SEQ ID NO: 1.

4. The enzyme for use according to any one of claims 1 to 3, wherein the enzyme consists of the amino acid sequence of SEQ ID NO: 1.

5. The enzyme according to any one of claims 1 to 4 for use in a method of preventing or treating a condition or disease that is associated with increased levels of biogenic amines in a subject.

6. The enzyme for use according to claim 5, wherein the increased levels of biogenic amines are due to the ingestion of said biogenic amines.

7. The enzyme for use according to claim 5 or claim 6, wherein the condition or disease that is associated with increased levels of biogenic amines is selected from the group consisting of allergies, acute and chronic allergic diseases, allergic reactions, allergy-like reactions, itching (pruritus), diarrhea, redness (erubescence), vomiting (emesis), acute and chronic biogenic amine poisoning, hypotonia, difficulty of breathing, biogenic amine intolerance, anaphylaxis, anaphylactic shock, acute and chronic urticaria, asthma, hay fever, allergic rhinitis, allergic conjunctivitis, headache, migraine, atopic dermatitis, mastocytosis, mast cell activation syndrome (MCAS), pre-eclampsia, hyperemesis gravidarum, pre-term labor, peptic ulcers, acid reflux, sepsis, fibromyalgia, chronic fatigue syndrome, and spondylitis.

8. Use of an enzyme having diamine oxidase activity in the production of a biogenic amine-depleted product, wherein said enzyme is as defined in any one of claims 1 to 4.

9. A method for the production of a biogenic amine-depleted product, comprising the step of contacting (i) a biogenic amine-containing product, and/or (ii) a biogenic amine-containing intermediate product of said product, with an enzyme having diamine oxidase activity under conditions and for a duration of time suitable to degrade a biogenic amine present in said product and/or in said intermediate product of said product, wherein said enzyme is as defined in any one of claims 1 to 4.

10. The use of claim 8 or the method of claim 9, wherein the product is selected from the group consisting of foodstuffs and feedstuffs.

11. The use of claim 8 or the method of claim 9, wherein the product is a foodstuff, selected from the group consisting of fermented foodstuffs, cheeses, sauerkraut, sausages, wine, chocolate, yeast extracts, fish, fish products, raw meats, and fresh milk.

12. A functional food or dietary supplement, comprising an enzyme having diamine oxidase activity, wherein said enzyme is as defined in any one of claims 1 to 4.

13. The enzyme for use according to any one of claims 1 to 7, the use according to any one of claims 8, 10, or 11, the method according to any one of claims 9 to 11, or the functional food or dietary supplement according to claim 12, wherein the biogenic amine is selected from the group consisting of histamine, tyramine, putrescine, cadaverine, agmatine, spermidine, and tryptamine.

14. The enzyme for use according to any one of claims 1 to 7, the use according to any one of claims 8, 10, or 11, the method according to any one of claims 9 to 11, or the functional food or dietary supplement according to claim 12, wherein the biogenic amine is selected from the group consisting of histamine, tyramine, putrescine, and cadaverine.

15. The enzyme for use according to any one of claims 1 to 7, the use according to any one of claims 8, 10, or 11, the method according to any one of claims 9 to 11, or the functional food or dietary supplement according to claim 12, wherein the biogenic amine is histamine.
